# EUROPEAN PATENT APPLICATION

(11) **EP 4 494 658 A1**
(43) Date of publication of application: **22.01.2025**
(21) Application number: 23769715.6
(22) Date of filing: 13.03.2023
(51) Int. Cl.: A61K 47/68, C07K 16/28, A61K 39/395, A61K 38/08, A61P 35/00

(54) **ANTIBODY-DRUG CONJUGATE AND USE THEREOF**

(30) Priority: 14.03.2022 CN 202210246477
(71) Applicant: Shanghai Huaota Biopharmaceutical Co., Ltd., Shanghai 201203 (CN); Huabo Biopharm (Shanghai) Co., Ltd., Shanghai 201203 (CN)
(72) Inventor: XU, Jingen, Shanghai 201203 (CN); ZHU, Xiangyang, Shanghai 201203 (CN); ZHAN, Yifan, Shanghai 201203 (CN); LI, Xue, Shanghai 201203 (CN); CHEN, Shi, Shanghai 201203 (CN); XIA, Simin, Shanghai 201203 (CN); PAN, Xianfei, Shanghai 201203 (CN)
(74) Representative: Weickmann & Weickmann PartmbB
(86) International application number: PCT/CN2023/081100
(87) International publication number: WO 2023/174213

(57) **Abstract**

An antibody-drug conjugate, comprising an antibody targeting CD73 or an antigen-binding fragment thereof. The antibody targeting CD73 or the antigen-binding fragment thereof comprises HCDR3, the HCDR3 comprising an amino acid sequence as set forth in SEQ ID NO: 3. Also provided are a preparation method for the antibody-drug conjugate and use thereof.

## Description

### TECHNICAL FIELD

The present application relates to the field of biomedicine and particularly to an antibody-drug conjugate and use thereof.

### BACKGROUND

Cluster of differentiation 73 (CD73) is a surface glycoprotein anchored to the exterior of the cell membrane via glycosylphosphatidylinositol (GPI). CD73 has been reported to be expressed in many different cancers. In addition, the expression of CD73 in cancer is associated with increased proliferation, migration, neovascularization, invasiveness, metastasis, and shorter patient survival. In addition to being effective against immunosuppression, CD73 is abnormally activated and expressed in tumor tissues, and compared to other targets, antibodies targeting CD73 can be rapidly and abundantly internalized. It can be seen that CD73 is an important target for antibody-drug conjugates (ADCs).

An antibody-drug conjugate generally comprises three moieties: an antigen-binding moiety, a small-molecule drug, and a linker that conjugates the antigen-binding moiety to the drug. There is an urgent need to obtain highly specific and safe antibody-drug conjugates against human CD73.

### SUMMARY

In one aspect, the present application provides an antibody-drug conjugate, comprising a CD73-targeting antibody or an antigen-binding fragment thereof, wherein the CD73-targeting antibody or the antigen-binding fragment thereof comprises HCDR3, the HCDR3 comprising the amino acid sequence as set forth in SEQ ID NO: 3.

In certain embodiments, the CD73-targeting antibody or the antigen-binding fragment thereof comprises HCDR2, the HCDR2 comprising the amino acid sequence as set forth in SEQ ID NO: 2. In certain embodiments, the CD73-targeting antibody or the antigen-binding fragment thereof comprises HCDR1, the HCDR1 comprising the amino acid sequence as set forth in SEQ ID NO: 1. In certain embodiments, the CD73-targeting antibody or the antigen-binding fragment thereof comprises LCDR3, the LCDR3 comprising the amino acid sequence as set forth in SEQ ID NO: 6. In certain embodiments, the CD73-targeting antibody or the antigen-binding fragment thereof comprises LCDR2, the LCDR2 comprising the amino acid sequence as set forth in SEQ ID NO: 5. In certain embodiments, the CD73-targeting antibody or the antigen-binding fragment thereof comprises LCDR1, the LCDR1 comprising the amino acid sequence as set forth in SEQ ID NO: 4. In certain embodiments, the CD73-targeting antibody or the antigen-binding fragment thereof comprises H-FR1, the C-terminus of the H-FR1 being linked directly or indirectly to the N-terminus of the HCDR1, and the H-FR1 comprising the amino acid sequence as set forth in SEQ ID NO: 7, SEQ ID NO: 15, or SEQ ID NO: 19.

In certain embodiments, the CD73-targeting antibody or the antigen-binding fragment thereof comprises H-FR2, the H-FR2 being located between the HCDR1 and the HCDR2, and the H-FR2 comprising the amino acid sequence as set forth in SEQ ID NO: 8, SEQ ID NO: 16, or SEQ ID NO: 21.

In certain embodiments, the CD73-targeting antibody or the antigen-binding fragment thereof comprises H-FR3, the H-FR3 being located between the HCDR2 and the HCDR3, and the H-FR3 comprising the amino acid sequence as set forth in SEQ ID NO: 9, SEQ ID NO: 17, SEQ ID NO: 20, or SEQ ID NO: 22.

In certain embodiments, the CD73-targeting antibody or the antigen-binding fragment thereof comprises H-FR4, the N-terminus of the H-FR4 being linked to the C-terminus of the HCDR3, and the H-FR4 comprising the amino acid sequence as set forth in SEQ ID NO: 10 or SEQ ID NO: 18. In certain embodiments, the CD73-targeting antibody or the antigen-binding fragment thereof comprises L-FR1, the C-terminus of the L-FR1 being linked directly or indirectly to the N-terminus of the LCDR1, and the L-FR1 comprising the amino acid sequence as set forth in SEQ ID NO: 11 or SEQ ID NO: 23.

In certain embodiments, the CD73-targeting antibody or the antigen-binding fragment thereof comprises L-FR2, the L-FR2 being located between the LCDR1 and the LCDR2, and the L-FR2 comprising the amino acid sequence as set forth in SEQ ID NO: 12 or SEQ ID NO: 24.

In certain embodiments, the CD73-targeting antibody or the antigen-binding fragment thereof comprises L-FR3, the L-FR3 being located between the LCDR2 and the LCDR3, and the L-FR3 comprising the amino acid sequence as set forth in SEQ ID NO: 13, SEQ ID NO: 25, SEQ ID NO: 27, or SEQ ID NO: 28.

In certain embodiments, the CD73-targeting antibody or the antigen-binding fragment thereof comprises L-FR4, the N-terminus of the L-FR4 being linked to the C-terminus of the LCDR3, and the L-FR4 comprising the amino acid sequence as set forth in SEQ ID NO: 14 or SEQ ID NO: 26.

In certain embodiments, the HCDR3 comprises the amino acid sequence as set forth in SEQ ID NO: 3, the HCDR2 comprises the amino acid sequence as set forth in SEQ ID NO: 2, and the HCDR1 comprises the amino acid sequence as set forth in SEQ ID NO: 1; and the LCDR3 comprises the amino acid sequence as set forth in SEQ ID NO: 6, the LCDR2 comprises the amino acid sequence as set forth in SEQ ID NO: 5, and the LCDR1 comprises the amino acid sequence as set forth in SEQ ID NO: 4.

In certain embodiments, the CD73-targeting antibody or the antigen-binding fragment thereof comprises VH, the VH comprising the amino acid sequence as set forth in SEQ ID NO: 29, SEQ ID NO: 31, SEQ ID NO: 33, or SEQ ID NO: 35.

In certain embodiments, the CD73-targeting antibody or the antigen-binding fragment thereof comprises VL, the VL comprising the amino acid sequence as set forth in SEQ ID NO: 30, SEQ ID NO: 32, SEQ ID NO: 34, or SEQ ID NO: 36.

In certain embodiments, the CD73-targeting antibody or the antigen-binding fragment thereof comprises any one of the groups of VH and VL selected from: the VH comprising the amino acid sequence as set forth in SEQ ID NO: 29 and the VL comprising the amino acid sequence as set forth in SEQ ID NO: 30; the VH comprising the amino acid sequence as set forth in SEQ ID NO: 31 and the VL comprising the amino acid sequence as set forth in SEQ ID NO: 32; the VH comprising the amino acid sequence as set forth in SEQ ID NO: 33 and the VL comprising the amino acid sequence as set forth in SEQ ID NO: 34; the VH comprising the amino acid sequence as set forth in SEQ ID NO: 35 and the VL comprising the amino acid sequence as set forth in SEQ ID NO: 36; the VH comprising the amino acid sequence as set forth in SEQ ID NO: 33 and the VL comprising the amino acid sequence as set forth in SEQ ID NO: 36; and the VH comprising the amino acid sequence as set forth in SEQ ID NO: 35 and the VL comprising the amino acid sequence as set forth in SEQ ID NO: 34.

In certain embodiments, the CD73-targeting antibody or the antigen-binding fragment thereof comprises an antibody heavy chain constant region.

In certain embodiments, the antibody heavy chain constant region is derived from a human antibody heavy chain constant region.

In certain embodiments, the antibody heavy chain constant region is derived from a human IgG heavy chain constant region.

In certain embodiments, the antibody heavy chain constant region is derived from a human IgG1 heavy chain constant region.

In certain embodiments, the CD73-targeting antibody or the antigen-binding fragment thereof comprises an antibody light chain constant region.

In certain embodiments, the antibody light chain constant region is derived from a human Igκ constant region.

In certain embodiments, the CD73-targeting antibody or the antigen-binding fragment thereof comprises an antibody or an antigen-binding fragment thereof.

In certain embodiments, the antigen-binding fragment includes a Fab, a Fab', an Fv fragment, an F(ab')2, an F(ab)2, an scFv, a di-scFv, and/or a dAb.

In certain embodiments, the antibody is selected from one or more of the group consisting of: a monoclonal antibody, a chimeric antibody, a humanized antibody, and a fully human antibody.

In certain embodiments, the CD73 includes human CD73 and/or monkey CD73.

In certain embodiments, the antibody-drug conjugate is capable of preventing or reducing activation of the CD73.

In certain embodiments, the antibody-drug conjugate is capable of inhibiting the enzymatic activity of the CD73.

In certain embodiments, the antibody-drug conjugate is capable of inducing endocytosis of the CD73 on a cell surface.

In certain embodiments, the antibody-drug conjugate comprises a structure represented by formula 1: M-(L1)a-(L2)b-D (formula 1), wherein M represents the CD73-targeting antibody or the antigen-binding fragment thereof of the present application; L1 represents a linker linking to M, and L2 represents a linker linking to D; each b is independently selected from 0-10; D represents a drug.

In certain embodiments, the L1 and/or the L2 are/is selected from the group consisting of: a cleavable linker, a non-cleavable linker, a hydrophilic linker, a hydrophobic linker, a charged linker, an uncharged linker, and a dicarboxylic acid-based linker.

In certain embodiments, the L1 is linked to the M by a sulfhydryl group, an azido group, or an amide group on the M.

In certain embodiments, the L1 is capable of reacting with the sulfhydryl group to achieve linkage. In certain embodiments, the L1 is selected from the group consisting of: 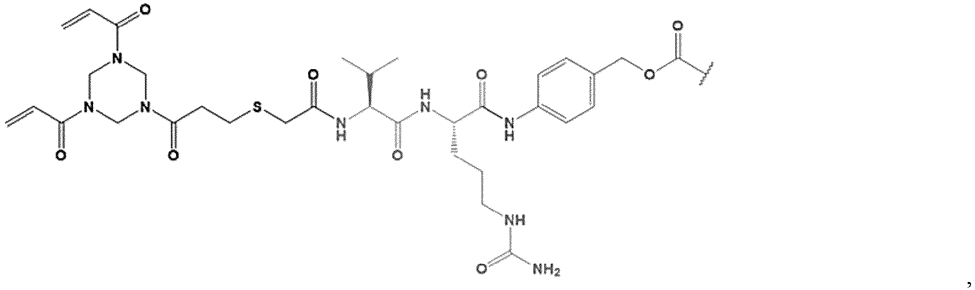 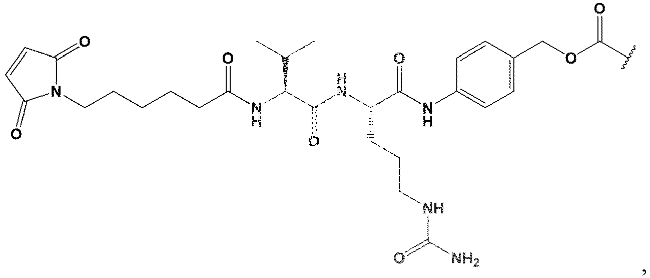 and

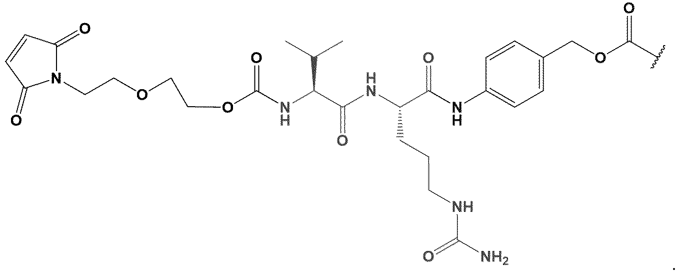

In certain embodiments, the L2 is selected from the group consisting of: a polypeptide, N-succinimidyl-3-(2-pyridyldithio)propionate (SPDP), N-succinimidyl 4-(2-pyridyldithio)pentanoate (SPP), N-succinimidyl 4-(2-pyridyldithio)butyrate (SPDB), N-succinimidyl-4-(2-pyridyldithio)-2-sulfobutyrate (sulfo-SPDB), N-succinimidyl iodoacetate (SIA), N-succinimidyl (4-iodoacetyl)aminobenzoate (SIAB), maleimide PEG NHS, succinimidyl 4-(N-maleimidomethyl)cyclohexane-1-carboxylate (SMCC), sulfosuccinimidyl 4-(N-maleimidomethyl)cyclohexane-1-carboxylate (sulfo-SMCC), and 2,5-dioxopyrrolidin-1-yl 17-(2,5-dioxo-2,5-dihydro-1H-pyrrol-1-yl)-5,8,11,14-tetraoxo-4,7,10,13-tetraazaoctadecan-1-oate (CX1-1).

In certain embodiments, the L2 is:

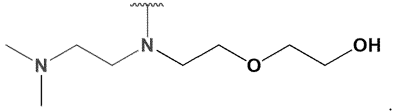

In certain embodiments, the L1-L2 is selected from the group consisting of: 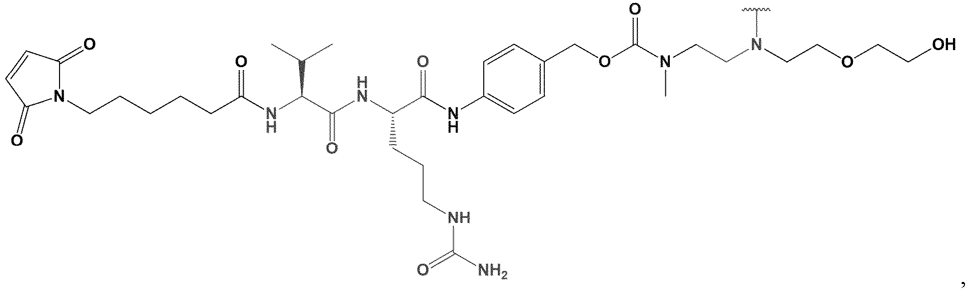 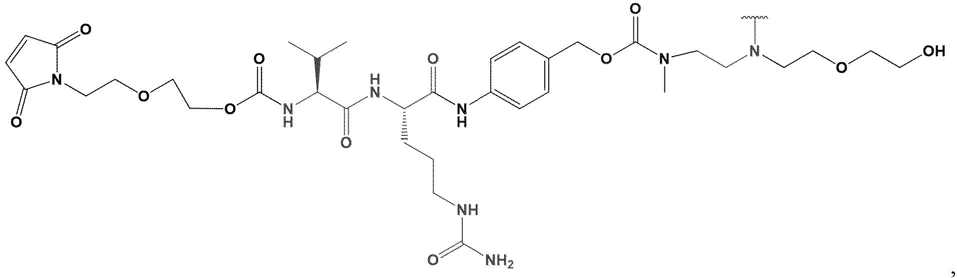 and

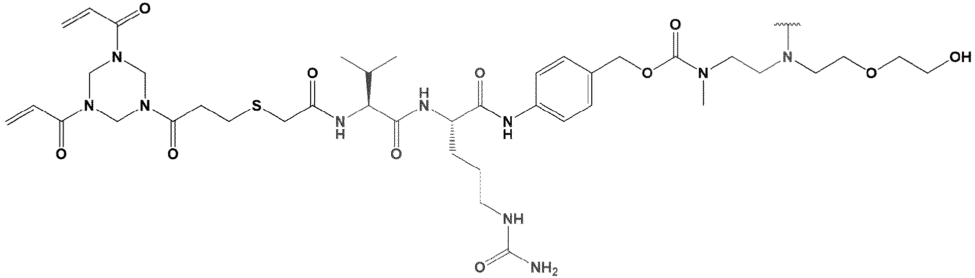

In certain embodiments, the drug has an ability to kill tumor cells, and/or an ability to inhibit tumor cell growth.

In certain embodiments, the drug includes a small-molecule drug.

In certain embodiments, the drug is selected from the group consisting of: a V-ATPase inhibitor, a Bcl2 inhibitor, an MCL1 inhibitor, an HSP90 inhibitor, an IAP inhibitor, an mTor inhibitor, a microtubule stabilizer, a microtubule destabilizer, auristatin, dolastatin, a maytansinoid, MetAP (methionine aminopeptidase), an inhibitor of nuclear export of protein CRM1, a DPPIV inhibitor, a proteasome inhibitor, an inhibitor of phosphoryl transfer reactions in mitochondria, a protein synthesis inhibitor, a CDK2 inhibitor, a CDK9 inhibitor, a kinesin inhibitor, an HDAC inhibitor, a DNA damaging agent, a DNA alkylating agent, a DNA intercalator, a DNA minor groove binder, a DHFR inhibitor, a nucleoside analog, an HD AC inhibitor, an anthracycline, a NAMPT inhibitor, SN-38 or a derivative thereof, etoposide phosphate, nitrogen mustard, a proteasome inhibitor, a cytokine, a tubulysin B analog, and a Toll-like receptor agonist.

In certain embodiments, the drug is selected from the group consisting of: DM1, exatecan, Dxd, MMAE, SN-38, calicheamicin, anthracyclin-5G, DM4, microtubule inhibitor SHR153024, PNU-159682, eribulin, Tub196, Duo5 toxin, SN38, or derivatives thereof.

In certain embodiments, the (L1)ₐ-(L2)_{b}-D has a structure selected from the group consisting of: 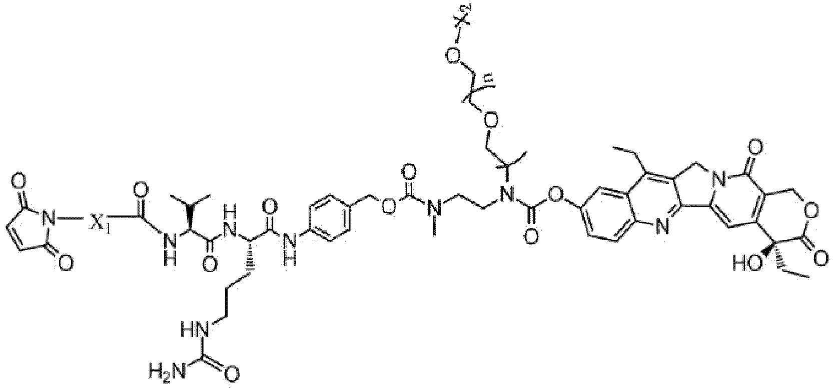 wherein
X₁ is an alkane chain or a PEG chain, and
X₂ is H or -C(O)NR¹R², and
R¹ is selected from hydrogen, halogen, hydroxyl, substituted or unsubstituted C₁₋₆ alkyl, substituted or unsubstituted C₁₋₆ hydroxyalkyl, substituted or unsubstituted C₁₋₆ aminoalkyl, substituted or unsubstituted C₁₋₆ alkoxy, substituted or unsubstituted C₁₋₆ alkanoyl, and a substituted or unsubstituted C₁₋₆ alkyl aldehyde group;
R² is selected from hydrogen and substituted or unsubstituted C₁₋₆ alkyl; each substituted C₁₋₆ alkyl is optionally substituted with 1-5 substituents independently selected from: halogen, hydroxyl, amino, carbonyl, carboxyl, 5- to 10-membered heteroaryl, and C₁₋₆ haloalkyl, wherein the 5- to 10-membered heteroaryl has 1-3 heteroatoms selected from oxygen, nitrogen, and sulfur; n is an integer greater than or equal to 1 and less than or equal to 20.

In certain embodiments, the (L1)ₐ-(L2)_{b}-D has a structure selected from the group consisting of: 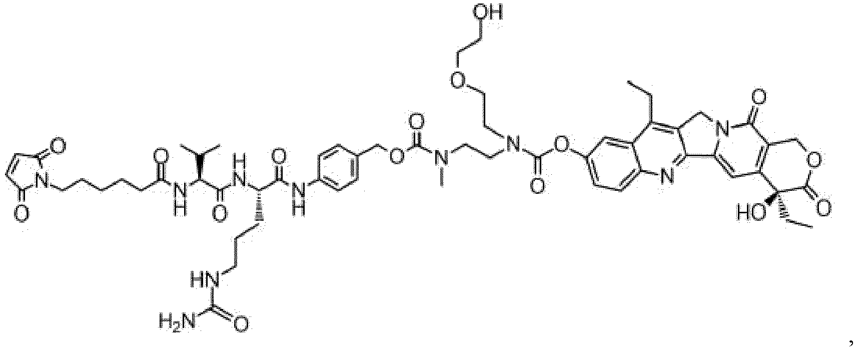 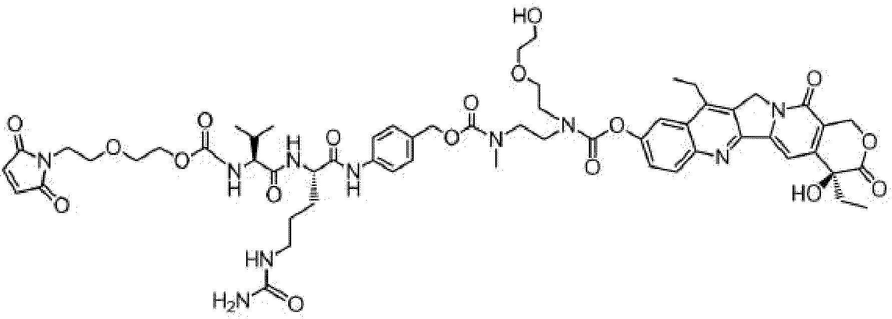 and

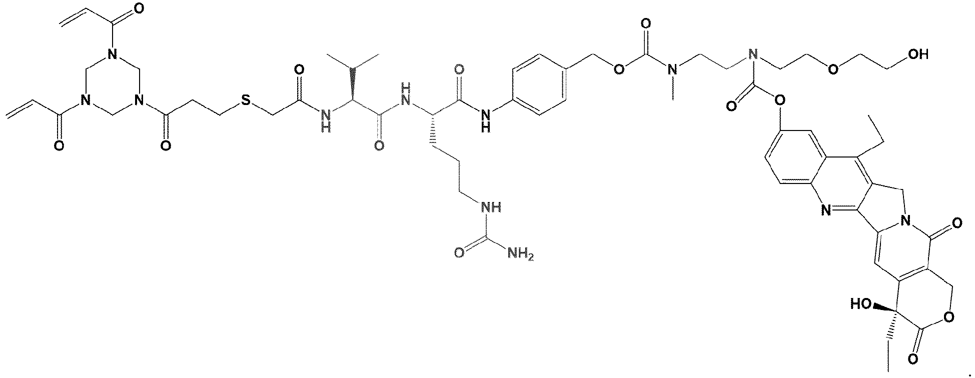

In certain embodiments, the antibody-drug conjugate has a structure selected from the group consisting of: 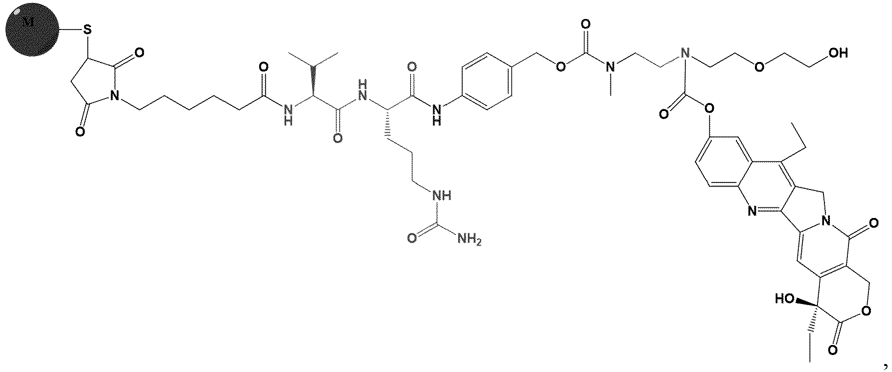 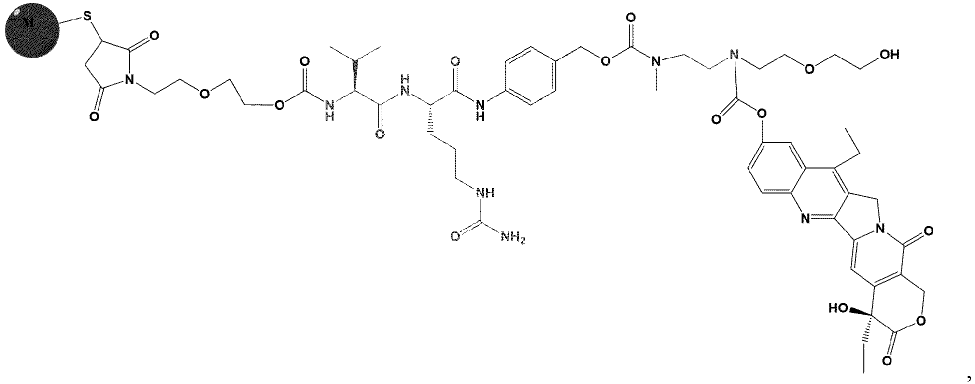 and

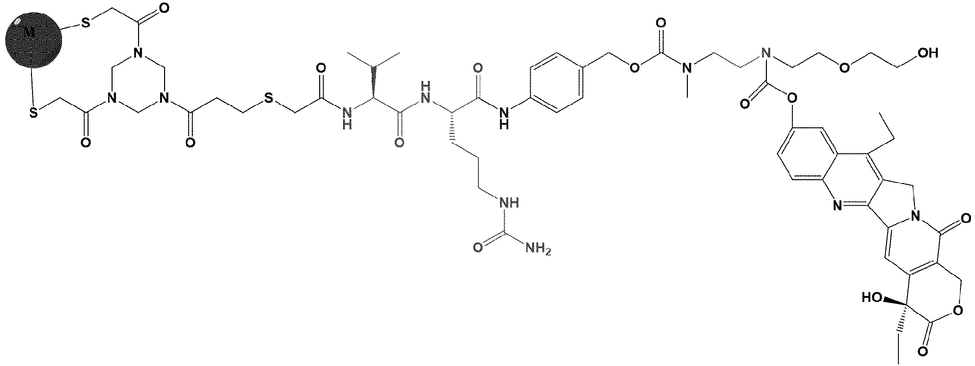

In certain embodiments, the antibody-drug conjugate has a drug-to-antibody ratio of about 1 to about 8.

In another aspect, the present application provides a compound that can be used for preparing the antibody-drug conjugate of the present application, which has a structure represented by formula 2: M-(L1)ₐ (formula 2), wherein M represents the CD73-targeting antibody or the antigen-binding fragment thereof of the present application; L1 represents a linker linking to M; a is selected from 0-10.

In certain embodiments, the L1 is selected from the group consisting of: a cleavable linker, a non-cleavable linker, a hydrophilic linker, a hydrophobic linker, a charged linker, an uncharged linker, and a dicarboxylic acid-based linker.

In certain embodiments, the L1 is linked to the M by a sulfhydryl group, an azido group, or an amide group on the M.

In certain embodiments, the L1 is capable of reacting with the sulfhydryl group to achieve linkage. In certain embodiments, the L1 is selected from the group consisting of: 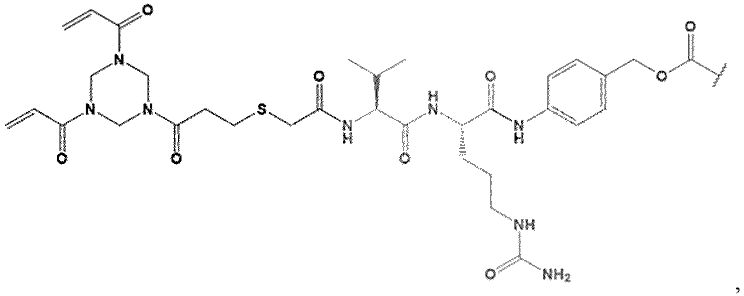 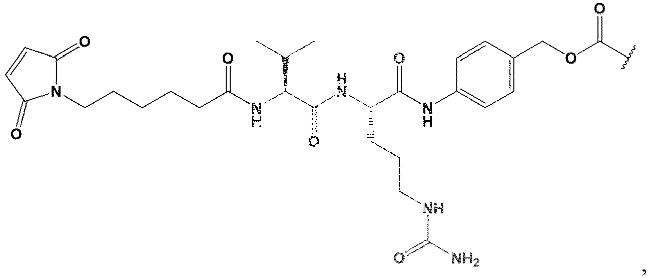 and

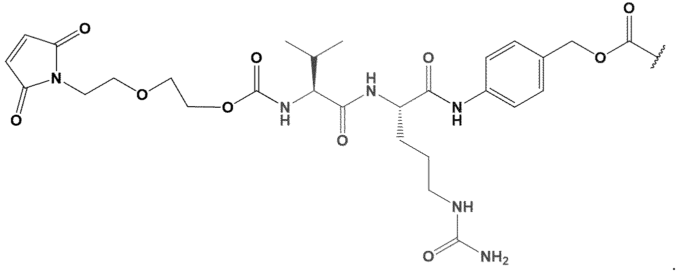

In another aspect, the present application provides a method for preparing the antibody-drug conjugate of the present application, comprising the following step: contacting the compound of the present application with the drug of the present application.

In another aspect, the present application provides a pharmaceutical composition, comprising the antibody-drug conjugate of the present application and optionally a pharmaceutically acceptable carri er.

In another aspect, the present application provides a method for modulating a tumor microenvironment in a subject, comprising the following step: administering to the subject the antibody-drug conjugate of the present application or the pharmaceutical composition of the present application.

In another aspect, the present application provides a method for modulating an immune response in a subject, comprising the following step: administering to the subject the antibody-drug conjugate of the present application or the pharmaceutical composition of the present application.

In another aspect, the present application provides use of the antibody-drug conjugate of the present application or the pharmaceutical composition of the present application in the preparation of a medicament, wherein the medicament is capable of preventing and/or treating a tumor.

In certain embodiments, the tumor includes a solid tumor and/or a non-solid tumor.

In certain embodiments, the tumor includes a CD73-mediated tumor.

In certain embodiments, the tumor includes pancreatic cancer and/or breast cancer.

Other aspects and advantages of the present application will be readily apparent to those skilled in the art from the following detailed description. Only exemplary embodiments of the present application have been shown and described in the following detailed description. As those skilled in the art will recognize, the content of the present application enables those skilled in the art to make changes in the specific embodiments disclosed without departing from the spirit and scope of the invention to which the present application relates. Accordingly, descriptions in the drawings and specification of the present application are only illustrative rather than restrictive.

### BRIEF DESCRIPTION OF THE DRAWINGS

The specific features of the invention to which the present application relates are as set forth in the appended claims. The features and advantages of the invention to which the present application relates may be more fully understood with reference to the exemplary embodiments and drawings described in detail below. The drawings are briefly described as follows:
FIGs. 1A-1D show structural schematic diagrams, HIC detection results, and DAR values of the antibody-drug conjugates of the present application.
FIG. 2 shows the assay results for the binding activity of the antibody-drug conjugates of the present application to a CD73 antigen on the cell membrane surface.
FIG. 3 shows the assay results for the inhibition of the enzymatic activity of a recombinant CD73 protein by the antibody-drug conjugates of the present application.
FIG. 4 shows the endocytosis efficiency of the antibody-drug conjugates of the present application in inducing endocytosis of CD73 on the cell membrane surface.
FIG. 5 shows the killing curves of the antibody-drug conjugates of the present application against tumor cells not expressing CD73.
FIG. 6 shows the killing curves of the antibody-drug conjugates of the present application against tumor cells highly expressing CD73.
FIG. 7 shows the anti-tumor effects of the antibody-drug conjugates of the present application on a CD73-expressing pancreatic cancer animal model.
FIG. 8 shows the anti-tumor effects of the antibody-drug conjugates of the present application on a CD73-expressing breast cancer animal model.
FIG. 9 shows the endocytosis efficiency of different antigen-binding moieties in inducing endocytosis of CD73 on the cell membrane surface.
FIGs. 10A-10B show the RP-HPLC chromatograms of the antibody-drug conjugates of the present application.
FIG. 11 shows the anti-tumor effects of the antibody-drug conjugates of the present application on a CD73-expressing breast cancer animal model.

### DETAILED DESCRIPTION

The embodiments of the invention in the present application are described below with reference to specific examples, and other advantages and effects of the invention in the present application will be readily apparent to those skilled in the art from the disclosure of the present specification.

### Definitions of Terms

In the present application, the term "antibody-drug conjugate" generally refers to an ADC, i.e., a binding protein (e.g., an antibody or an antigen-binding fragment thereof) linked to one or more chemical drugs. The chemical drugs may be any therapeutic agents and/or cytotoxic agents. The antibody-drug conjugate may have anywhere from 1 to 8 drugs conjugated to the antibody; for example, it may include drug loaded species of 2, 4, 6, or 8. In the present application, the drug may include mitotic inhibitors, anti-tumor antibiotics, immunomodulating agents, vectors for gene therapy, alkylating agents, anti-angiogenic agents, anti-metabolites, boron-containing agents, chemoprotective agents, hormones, anti-hormone agents, corticosteroids, photoactive therapeutic agents, oligonucleotides, radionuclide agents, topoisomerase inhibitors, tyrosine kinase inhibitors, and/or radiosensitizers.

In the present application, the term "drug-to-antibody ratio" or "DAR" generally refers to the number of drugs linked to the antibody of the ADC. The DAR of an ADC may range from 1 to 8, or higher loads (e.g., 10) are also possible. The range of the DAR can depend on the number of linking sites on the antibody. In the present application, the DAR may be the number of drugs loaded onto an individual antibody. The DAR may also be an average or mean DAR of a group of ADCs.

In the present application, the term "CD73" is also referred to as "NT5E", "cluster of differentiation 73", "5'-nucleotidase (5'-NT)", or "extracellular 5'-nucleotidase". The term encompasses "full-length" unprocessed CD73, as well as any form of CD73 that results from cellular processing. In the present application, the term "CD73" includes full-length wild-type CD73 and mutants, fragments, variants, isotypes and homologs thereof. In certain embodiments, the CD73 may include human CD73. For example, the sequence for human CD73 can be found under the Uniprot accession number P21589. In the present application, the CD73 may comprise the amino acid sequence as set forth in SEQ ID NO: 40.

In the present application, the term "CDR", also known as "complementarity-determining region", generally refers to a region in an antibody variable domain, which is highly variable in sequence and/or forms a structurally defined loop. Generally, an antibody comprises six CDRs; three in the VH (HCDR1, HCDR2, HCDR3) and three in the VL (LCDR1, LCDR2, LCDR3). In certain embodiments, a naturally occurring camelid antibody consisting of only heavy chains is also capable of functioning normally and stably in the absence of light chains. See, e.g., Hamers-Casterman et al., Nature 363: 446-448 (1993); Sheriff et al., Nature Struct. Biol. 3: 733-736 (1996). The CDRs of the antibody can be determined by a variety of coding systems, such as CCG, Kabat, Chothia, IMGT, a combination of Kabat/Chothia, and the like. These coding systems are known in the art and can be referenced in detail at, for example, http://www.bioinf.org.uk/abs/index.html#kabatnum. For example, the amino acid sequence of the antigen-binding protein can be numbered according to the IMGT numbering scheme (IMGT, the international ImMunoGeneTics information system@imgt.cines.fr; http://imgt.cines.fr; Lefranc et al., 1999, Nucleic Acids Res. 27: 209-212; Ruiz et al., 2000 Nucleic Acids Res. 28: 219-221; Lefranc et al., 2001, Nucleic Acids Res. 29: 207-209; Lefranc et al., 2003, Nucleic Acids Res. 31: 307-310; Lefranc et al., 2005, Dev Comp Immunol 29: 185-203). For example, the CDRs of the antigen-binding protein can be determined according to the Kabat numbering system (see, e.g., Kabat EA & Wu TT (1971) Ann NY Acad Sci 190: 382-391 and Kabat EA et al., (1991) Sequences of Proteins of Immunological Interest, Fifth Edition, U.S. Department of Health and Human Services, NIH Publication No. 91-3242).

In the present application, the term "FR" generally refers to a more highly conserved portion of an antibody variable domain, which is referred to as a framework region. Generally, the variable domains of natural heavy and light chains each comprise four FRs, namely four in the VH (H-FR1, H-FR2, H-FR3, and H-FR4) and four in the VL (L-FR1, L-FR2, L-FR3, and L-FR4).

In the present application, the terms "variable domain" and "variable region" are used interchangeably and generally refer to a portion of an antibody heavy and/or light chain. The variable domains of the heavy and light chains can be referred to as "V_{H}" and "V_{L}", respectively (or "VH" and "VL", respectively). These domains are generally the most variable portions of an antibody (relative to other antibodies of the same type) and comprise the antigen-binding sites.

In the present application, the term "variable" generally means that certain segments of the variable domains may differ greatly in sequence among antibodies. The variable domain mediates antigen binding and defines the specificity of a specific antibody for its specific antigen. However, the variability is not evenly distributed throughout the entire variable domain. It is generally concentrated in three segments called hypervariable regions (CDRs or HVRs) in the light and heavy chain variable domains. The more highly conserved portions of the variable domains are referred to as framework regions (FRs). The variable domains of natural heavy and light chains each comprise four FRs largely in a β-sheet configuration. The FRs are connected by three CDRs to form a loop connection, and in some cases to form part of a β-sheet structure. The CDRs in each chain are held closely together by the FRs, and, together with the CDRs from the other chain, contribute to the formation of the antigen-binding sites of the antibody (see Kabat et al., Sequences of Immunological Interest, Fifth Edition, National Institute of Health, Bethesda, Md. (1991)).

In the present application, the term "antibody" generally refers to an immunoglobulin or a fragment thereof or a derivative thereof, and encompasses any polypeptide that comprises an antigen-binding site, regardless of whether it is produced *in vitro* or *in vivo.* The term includes, but is not limited to, polyclonal, monoclonal, monospecific, multispecific, nonspecific, humanized, single-chain, chimeric, synthetic, recombinant, hybrid, mutated, and grafted antibodies. Unless otherwise modified by the term "intact", as in "intact antibody", for the purposes of the present invention, the term "antibody" also includes antibody fragments such as Fab, F(ab')₂, Fv, scFv, Fd, dAb, and other antibody fragments that retain antigen-binding functions (e.g., specifically binding to human CD73). Generally, such fragments should include an antigen-binding domain. The basic 4-chain antibody unit is a heterotetrameric glycoprotein consisting of two identical light (L) chains and two identical heavy (H) chains. An IgM antibody consists of 5 basic heterotetrameric units along with an additional polypeptide called the J chain, and contains 10 antigen-binding sites. In contrast, an IgA antibody comprises 2-5 basic 4-chain units that can polymerize with the J chain to form multivalent combinations. For IgG, the 4-chain unit typically has a molecular weight of about 150,000 daltons. Each L chain is linked to an H chain by one covalent disulfide bond, while the two H chains are linked to each other by one or more disulfide bonds depending on the H chain isotype. Each H and L chain also has regularly spaced intrachain disulfide bonds. Each H chain has a variable domain (VH) at the N-terminus, followed by three (for each of the α and γ chains) constant domains (CH) or four (for the µ and ε isotypes) CH domains. Each L chain has a variable domain (VL) at the N-terminus and a constant domain at its other end. VL corresponds to VH, and CL corresponds to the first constant domain of the heavy chain (CH1). Specific amino acid residues are considered to form an interface between the light and heavy chain variable domains. VH and VL pair together to form a single antigen-binding site. For the structures and properties of different classes of antibodies, see, e.g., Basic and Clinical Immunology, 8th Edition, Daniel P. Sties, Abba I. Terr and Tristram G. Parsolw (eds), Appleton & Lange, Norwalk, Conn., 1994, page 71 and chapter 6. The L chains from any vertebrate species can be divided into one of two distinctly different types, κ and λ, based on the amino acid sequences of constant domains thereof. Immunoglobulins can be divided into different classes or isotypes, based on the amino acid sequences of the constant domains of the heavy chains (CH). There are currently five classes of immunoglobulins: IgA, IgD, IgE, IgG, and IgM, which have heavy chains designated α, δ, ε, γ, and µ, respectively.

In the present application, the term "antigen-binding fragment" generally refers to one or more fragments having the ability to specifically bind to an antigen (e.g., CD73). In the present application, the antigen-binding fragment may include a Fab, a Fab', an F(ab)₂, an Fv fragment, an F(ab')₂, an scFv, a di-scFv, and/or a dAb.

In the present application, the term "Fab" generally refers to an antigen-binding fragment of an antibody. As described above, an intact antibody may be digested with papain. The antibody is digested with papain to produce two identical antigen-binding fragments, namely "Fab" fragments, and a residual "Fc" fragment (i.e., the Fc region, supra). The Fab fragment may consist of one intact L chain and the variable region of one heavy chain along with the first constant region (C_{H}1) of that H chain (V_{H}).

In the present application, the term "Fab' fragment" generally refers to a monovalent antigen-binding fragment of a human monoclonal antibody, which is slightly larger than the Fab fragment. For example, the Fab' fragment may include the entire light chain, the entire heavy chain variable region, and all or part of the first and second constant regions of the heavy chain. For example, the Fab' fragment may also include part or all of the 220-330 amino acid residues of the heavy chain.

In the present application, the term "F(ab')2" generally refers to an antibody fragment produced by pepsin digestion of an intact antibody. The F(ab')2 fragment contains two Fab fragments held together by disulfide bonds and part of a hinge region. F(ab')2 fragments have divalent antigen-binding activity and are capable of cross-linking antigens.

In the present application, the term "Fv fragment" generally refers to a monovalent antigen-binding fragment of a human monoclonal antibody, which comprises all or part of the heavy chain variable region and the light chain variable region, and lacks the heavy chain constant region and the light chain constant region. The heavy chain variable region and the light chain variable region include, for example, CDRs. For example, an Fv fragment comprises all or part of the amino-terminal variable regions of about 110 amino acids in the heavy and light chains.

In the present application, the term "scFv" generally refers to a fusion protein comprising at least one antibody fragment containing a light chain variable region and at least one antibody fragment containing a heavy chain variable region, wherein the light and heavy chain variable regions are contiguously linked (e.g., via a synthetic linker such as a short flexible polypeptide linker), and are capable of being expressed as a single-chain polypeptide, with the scFv retaining the specificity of the intact antibody from which it is derived. Unless otherwise specified, the scFv, as used herein, may have the VL and VH variable regions in any order (e.g., relative to the N-terminus and C-terminus of the polypeptide), and may comprise VL-linker-VH or VH-linker-VL.

In the present application, the term "dAb" generally refers to an antigen-binding fragment having a VH domain and a VL domain, or having a VH domain or a VL domain. See, e.g., Ward et al. (Nature, 1989 Oct 12; 341(6242): 544-6); Holt et al., Trends Biotechnol., 2003, 21(11): 484-490; and WO 06/030220, WO 06/003388, and other published patent applications by Domantis Ltd.

In the present application, the term "monoclonal antibody" generally refers to an antibody molecule preparation of a single-molecule composition. Monoclonal antibodies generally have high specificity for a single antigenic site. Moreover, unlike conventional polyclonal antibody formulations (which typically have different antibodies directed against different determinants), each monoclonal antibody is directed against a single determinant on the antigen. In addition to their specificity, the monoclonal antibodies are advantageous in that they can be synthesized by hybridoma culture without contamination by other immunoglobulins. The modifier "monoclonal" indicates the characteristic of an antibody obtained from a population of substantially homogeneous antibodies, and is not to be construed as requiring production of the antibody by any particular method. For example, the monoclonal antibodies used in the present application may be prepared in hybridoma cells, or may be prepared by the recombinant DNA method.

In the present application, the term "chimeric antibody" generally refers to an antibody in which the variable region is derived from one species and the constant region is derived from another species. Generally, the variable region is derived from an antibody ("parent antibody") of a laboratory animal such as a rodent, and the constant region is derived from a human antibody, such that the resulting chimeric antibody is less likely to induce an adverse immune response in a human individual as compared to the parent (e.g., mouse-derived) antibody.

In the present application, the term "humanized antibody" generally refers to an antibody in which some or all of the amino acids outside the CDRs of a non-human antibody (e.g., a mouse antibody) are replaced with corresponding amino acids derived from a human immunoglobulin. In the CDRs, small additions, deletions, insertions, substitutions, or modifications of amino acids may also be permissible, so long as they retain the ability of the antibody to bind to a specific antigen. A humanized antibody may optionally comprise at least a portion of a human immunoglobulin constant region. "Humanized antibody" retains antigen specificity similar to that of the original antibody. "Humanized" forms of non-human (e.g., murine) antibodies are chimeric antibodies minimally comprising sequences derived from non-human immunoglobulins. In certain cases, CDR residues of a human immunoglobulin (recipient antibody) may be replaced with CDR residues from a non-human species (donor antibody) (such as mouse, rat, rabbit, or non-human primate) having the desired properties, affinity, and/or capabilities. In certain cases, FR residues of a human immunoglobulin may be replaced with corresponding non-human residues. Furthermore, humanized antibodies may comprise amino acid modifications that are not present in the recipient antibody or in the donor antibody. These modifications may be made to further improve the properties of the antibodies, such as binding affinity.

The term "fully human antibody" generally refers to an antibody that comprises only human immunoglobulin protein sequences. A fully human antibody may contain mouse glycochains if it is produced in a mouse, in a mouse cell, or in a hybridoma derived from a mouse cell. Similarly, "mouse antibody" or "rat antibody" refers to an antibody that comprises only mouse or rat immunoglobulin sequences, respectively. Fully human antibodies can be generated in the human body or in transgenic animals with human immunoglobulin germline sequences through phage display or other molecular biology methods. Exemplary techniques that can be used to prepare antibodies are described in U.S. Patents: 6,150,584; 6,458,592; and 6,420,140. Other techniques, such as the use of libraries, are known in the art.

In the present application, the terms "polypeptide molecule", "polypeptide", and "peptide" are used interchangeably and generally refer to a polymer of amino acid residues. The term "fusion protein" generally refers to a polypeptide having at least two moieties covalently linked together, wherein each moiety may be a polypeptide having a different property. The property may be a biological property, such as activity *in vitro* or *in vivo.* The property may also be a simple chemical or physical property, such as binding to a target molecule, catalysis of a reaction, and the like. The two moieties may be directly linked by a single peptide bond or by a peptide linker.

In the present application, the term "nucleic acid molecule" generally refers to a nucleotide, deoxyribonucleotide or ribonucleotide of any length in its isolated form, or an analog thereof isolated from its natural environment or artificially synthesized.

In the present application, the term "vector" generally refers to a nucleic acid delivery vehicle in which a polynucleotide encoding a protein can be inserted to achieve expression of the protein. The vector can be introduced into host cells through transformation, transduction, or transfection, allowing the genetic substance elements it carries to be expressed in the host cells. By way of example, the vector may include: plasmids; phagemids; cosmids; artificial chromosomes, such as yeast artificial chromosomes (YACs), bacterial artificial chromosomes (BACs), or P1-derived artificial chromosomes (PACs); phages, such as λ phages or M13 phages; animal viruses; and the like. The types of animal viruses used as vectors may include retroviruses (including lentiviruses), adenoviruses, adeno-associated viruses, herpes viruses (such as herpes simplex virus), poxviruses, baculoviruses, papillomaviruses, and papovaviruses (such as SV40). A vector may comprise a variety of elements that control expression, including promoter sequences, transcription initiation sequences, enhancer sequences, selection elements, and reporter genes. In addition, the vector may also comprise a replication initiation site. The vector may further comprise components that assist its entry into cells, such as viral particles, liposomes, or protein shells, but are not limited to these substances.

In the present application, the term "cell" generally refers to a single cell, cell line or cell culture that can be or has been a recipient of a subject's plasmid or vector, which includes the nucleic acid molecule of the present application or the vector of the present application. The cell may include progeny of a single cell. Due to natural, accidental, or intentional mutations, the progeny may not necessarily be identical to the original parent cell (in terms of the morphology of the total DNA complement or the genome). The cell may include cells transfected *in vitro* with the vector of the present application. The cells may be bacterial (e.g., E. coli) cells, yeast cells, or other eukaryotic cells, such as COS cells, Chinese hamster ovary (CHO) cells, CHO-K1 cells, LNCAP cells, HeLa cells, HEK293 cells, COS-1 cells, or NS0 cells. In certain embodiments, the cell is a mammalian cell. In certain embodiments, the mammalian cell is an HEK293 cell.

In the present application, the 20 conventional amino acids and their abbreviations follow conventional usage. See Immunology A Synthesis (2nd Edition, E. S. Golub and D. R. Gren, Eds., Sinauer Associates, Sunderland, Mass. (1991)), which is incorporated herein by reference.

In the present application, the term "linker" generally refers to a chemical moiety that is bifunctional or multifunctional and is used to link the bispecific antibody or the antigen-binding fragment thereof of the present application to the drug of the present application. The linker may comprise one conjugation component or may comprise multiple conjugation components.

In the present application, the term "tumor" generally refers to a physiological condition in mammals that is typically characterized by unregulated cell growth. The tumor comprises one or more cancerous cells. The tumor may include a solid tumor and/or a non-solid tumor.

In the present application, the term "tumor microenvironment" refers generally to the environment in which a tumor exists, which includes the non-cellular area within the tumor and the area directly outside the tumor tissue but not belonging to the intracellular compartment of the cancer cell itself. The tumor and the tumor microenvironment are closely related and can interact constantly. For example, a tumor can change the tumor microenvironment, and the tumor microenvironment can affect the growth and spread of the tumor. Generally, the tumor microenvironment has a low pH in the range of 5.8 to 7.0. The tumor microenvironment may have a lower concentration of glucose and other nutrients, but a higher concentration of lactic acid. Furthermore, the temperature of the tumor microenvironment may be 0.3 to 1 °C higher than the normal physiological temperature. The tumor microenvironment has been discussed in Gillies et al., "MRI of the Tumor Microenvironment", Journal of Magnetic Resonance Imaging, vol. 16, pp. 430-450, 2002.

In the present application, the term "treatment" generally refers to both therapeutic treatments and prophylactic or preventative measures, which aim to prevent or slow down (mitigate) unwanted pathologic changes or disorders, such as the growth, development or spread of hyperproliferative conditions such as cancer. For the purposes of the present invention, beneficial or desired clinical results include, but are not limited to, alleviation of symptoms, reduction in the disease severity, stabilization (i.e., not worsening) of the disease state, delay or slowing down of the disease progression, amelioration or mitigation of the disease state, and recovery (whether partial or complete), whether detectable or undetectable. "Treatment" or "treating" may also refer to prolonging survival as compared to the expected survival without the treatment. Subjects in need of treatment include subjects who have long suffered from the disease or disorder, as well as subjects who are predisposed to developing the disease or disorder or subjects for whom the condition or disorder is to be prevented.

In the present application, the term "specifically binding" or "specific" generally refers to a measurable and reproducible interaction, such as binding between a target and an antibody, that may determine the presence of the target in the presence of a heterogeneous population of molecules (including biomolecules). For example, an antibody that specifically binds to a target (which may be an epitope) may be an antibody that binds to this target with greater affinity, avidity and ease, and/or for a longer duration than it binds to other targets. In certain embodiments, the antibody specifically binds to an epitope on the protein that is conserved in proteins of different species. In certain embodiments, specific binding may include, but is not required to be, exclusive binding.

In the present application, the term "subject" generally refers to a human or non-human animal, including but not limited to cats, dogs, horses, pigs, cows, sheep, rabbits, mice, rats, and monkeys. In the present application, the protein, polypeptide and/or amino acid sequence involved is also to be understood as including at least the following ranges: variants or homologs having the same or similar function as the protein or polypeptide.

In the present application, the variant may be, for example, a protein or polypeptide obtained by substitution, deletion, or addition of one or more amino acids in the amino acid sequence of the protein and/or polypeptide (e.g., an antibody or a fragment thereof that specifically binds to a CD73 protein). For example, the functional variant may include a protein or polypeptide with amino acid change through at least 1, such as 1-30, 1-20, or 1-10 (e.g., 1, 2, 3, 4, or 5) amino acid substitutions, deletions and/or insertions. The functional variant may substantially retain the biological properties of the protein or the polypeptide prior to the change (e.g., substitution, deletion, or addition). For example, the functional variant may retain at least 60%, 70%, 80%, 90%, or 100% of the biological activity (e.g., antigen-binding capacity) of the protein or the polypeptide prior to the change. For example, the substitution may be a conservative substitution.

In the present application, the homolog may be a protein or polypeptide comprising an amino acid sequence having at least about 85% (e.g., at least about 85%, about 90%, about 91%, about 92%, about 93%, about 94%, about 95%, about 96%, about 97%, about 98%, about 99% or higher) sequence homology to the amino acid sequence of the protein and/or the polypeptide (e.g., an antibody or a fragment thereof that specifically binds to a CD73 protein).

In the present application, the homology generally refers to the similarity, likeness, or association between two or more sequences. "Percent sequence homology" can be calculated by the following steps: comparing two sequences to be aligned in a comparison window; determining the number of positions at which nucleic acid bases (e.g., A, T, C, G, and I) or amino acid residues (e.g., Ala, Pro, Ser, Thr, Gly, Val, Leu, Ile, Phe, Tyr, Trp, Lys, Arg, His, Asp, Glu, Asn, Gln, Cys, and Met) are identical in the two sequences to give the number of matched positions; dividing the number of matched positions by the total number of positions in the comparison window (i.e., the window size); and multiplying the result by 100 to give a percent sequence homology. Alignment for determining the percent sequence homology can be achieved in a variety of ways known in the art, for example, using publicly available computer software such as BLAST, BLAST-2, ALIGN or Megalign (DNASTAR) software. Those skilled in the art can determine suitable parameters for alignment of the sequences, including any algorithm necessary to yield optimal alignment within a full-length sequence range or target sequence region being compared. The homology can also be determined by the following methods: FASTA and BLAST. For the description of the FASTA algorithm, see W. R. Pearson and D. J. Lipman, "Improved Tools for Biological Sequence Comparison" (Proc. Natl. Acad. Sci.), 85: 2444-2448, 1988; and D. J. Lipman and W. R. Pearson, "Rapid and Sensitive Protein Similarity Searches", Science, 227: 1435-1441, 1989. For description of the BLAST algorithm, see S. Altschul, W. Gish, W. Miller, E. W. Myers and D. Lipman, "ABasic Local Alignment Search Tool", Journal of Molecular Biology, 215: 403-410, 1990.

In the present application, the term "comprise" or "comprising" generally means including, summarizing, containing, or encompassing. In certain cases, the term also means "being" or "consisting of...".

In the present application, the term "about" generally means varying by 0.5%-10% above or below the stated value, for example, varying by 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the stated value.

### Detailed Description of the Invention

In one aspect, the present application provides:

### Antigen-binding protein

The CDRs of an antibody, also known as complementarity-determining regions, are part of the variable regions. The amino acid residues in these regions may be in contact with antigens or antigenic epitopes. The CDRs of the antibody can be determined by a variety of coding systems, such as CCG, Kabat, Chothia, IMGT, a combination of Kabat/Chothia, and the like. These coding systems are known in the art and can be referenced in detail at, for example, http://www.bioinf.org.uk/abs/index.html#kabatnum. Those skilled in the art can determine the CDRs using different coding systems based on the sequence and structure of the antibody. When using different coding systems, the CDRs may vary. In the present application, the CDR encompasses CDR sequences defined according to any CDR definition method; the CDR also encompasses variants thereof, which comprise one or more amino acid substitutions, deletions and/or additions, such as 1-30, 1-20, or 1-10 (for another example, 1, 2, 3, 4, 5, 6, 7, 8, or 9) amino acid substitutions, deletions and/or insertions in the amino acid sequence of the CDR; the CDR also encompasses homologs thereof, which may be amino acid sequences having at least about 85% (e.g., at least about 85%, about 90%, about 91%, about 92%, about 93%, about 94%, about 95%, about 96%, about 97%, about 98%, about 99%, or higher) sequence homology to the amino acid sequence of the CDR. In certain embodiments, the CDR is determined by the IMGT numbering scheme.

In the present application, the CD73-targeting antibody or the antigen-binding fragment thereof comprises HCDR3, wherein the HCDR3 may comprise the amino acid sequence as set forth in SEQ ID NO: 3.

In the present application, the CD73-targeting antibody or the antigen-binding fragment thereof may further comprise HCDR2, wherein the HCDR2 may comprise the amino acid sequence as set forth in SEQ ID NO: 2.

In the present application, the CD73-targeting antibody or the antigen-binding fragment thereof may further comprise HCDR1, wherein the HCDR1 may comprise the amino acid sequence as set forth in SEQ ID NO: 1.

In the present application, the CD73-targeting antibody or the antigen-binding fragment thereof may comprise HCDR3, HCDR2, and HCDR1. For example, in the CD73-targeting antibody or the antigen-binding fragment thereof, the HCDR3 may comprise the amino acid sequence as set forth in SEQ ID NO: 3, the HCDR2 may comprise the amino acid sequence as set forth in SEQ ID NO: 2, and the HCDR1 may comprise the amino acid sequence as set forth in SEQ ID NO: 1.

In the present application, the CD73-targeting antibody or the antigen-binding fragment thereof may comprise LCDR3, wherein the LCDR3 may comprise the amino acid sequence as set forth in SEQ ID NO: 6.

In the present application, the CD73-targeting antibody or the antigen-binding fragment thereof may further comprise LCDR2, wherein the LCDR2 may comprise the amino acid sequence as set forth in SEQ ID NO: 5.

In the present application, the CD73-targeting antibody or the antigen-binding fragment thereof may further comprise LCDR1, wherein the LCDR1 may comprise the amino acid sequence as set forth in SEQ ID NO: 4.

In the present application, the CD73-targeting antibody or the antigen-binding fragment thereof may comprise LCDR3, LCDR2, and LCDR1. For example, in the CD73-targeting antibody or the antigen-binding fragment thereof, the LCDR3 may comprise the amino acid sequence as set forth in SEQ ID NO: 6, the LCDR2 may comprise the amino acid sequence as set forth in SEQ ID NO: 5, and the LCDR1 may comprise the amino acid sequence as set forth in SEQ ID NO: 4.

In the present application, the CD73-targeting antibody or the antigen-binding fragment thereof may comprise HCDR3, HCDR2, HCDR1, LCDR3, LCDR2, and LCDR1. For example, in the CD73-targeting antibody or the antigen-binding fragment thereof of the present application, the HCDR3 may comprise the amino acid sequence as set forth in SEQ ID NO: 3, the HCDR2 may comprise the amino acid sequence as set forth in SEQ ID NO: 2, the HCDR1 may comprise the amino acid sequence as set forth in SEQ ID NO: 1, the LCDR3 may comprise the amino acid sequence as set forth in SEQ ID NO: 6, the LCDR2 may comprise the amino acid sequence as set forth in SEQ ID NO: 5, and the LCDR1 may comprise the amino acid sequence as set forth in SEQ ID NO: 4.

In the present application, the CD73-targeting antibody or the antigen-binding fragment thereof may comprise H-FR1, wherein the C-terminus of the H-FR1 may be linked directly or indirectly to the N-terminus of the HCDR1, and the H-FR1 may comprise the amino acid sequence as set forth in SEQ ID NO: 7, SEQ ID NO: 15, or SEQ ID NO: 19.

In the present application, the CD73-targeting antibody or the antigen-binding fragment thereof may comprise H-FR2, wherein the H-FR2 may be located between the HCDR1 and the HCDR2, and the H-FR2 may comprise the amino acid sequence as set forth in SEQ ID NO: 8, SEQ ID NO: 16, or SEQ ID NO: 21.

In the present application, the CD73-targeting antibody or the antigen-binding fragment thereof may comprise H-FR3, wherein the H-FR3 may be located between the HCDR2 and the HCDR3, and the H-FR3 may comprise the amino acid sequence as set forth in SEQ ID NO: 9, SEQ ID NO: 17, SEQ ID NO: 20, or SEQ ID NO: 22.

In the present application, the CD73-targeting antibody or the antigen-binding fragment thereof may comprise H-FR4, wherein the N-terminus of the H-FR4 may be linked to the C-terminus of the HCDR3, and the H-FR4 may comprise the amino acid sequence as set forth in SEQ ID NO: 10 or SEQ ID NO: 18.

In the present application, the antigen-binding protein may comprise H-FR1, H-FR2, H-FR3, and H-FR4. For example, the H-FR1, the H-FR2, the H-FR3, and the H-FR4 of the CD73-targeting antibody or the antigen-binding fragment thereof may comprise the amino acid sequences as set forth in SEQ ID NO: 7, SEQ ID NO: 8, SEQ ID NO: 9, and SEQ ID NO: 10, respectively. For example, the H-FR1, the H-FR2, the H-FR3, and the H-FR4 of the CD73-targeting antibody or the antigen-binding fragment thereof may comprise the amino acid sequences as set forth in SEQ ID NO: 15, SEQ ID NO: 16, SEQ ID NO: 17, and SEQ ID NO: 18, respectively. For example, the H-FR1, the H-FR2, the H-FR3, and the H-FR4 of the CD73-targeting antibody or the antigen-binding fragment thereof may comprise the amino acid sequences as set forth in SEQ ID NO: 19, SEQ ID NO: 16, SEQ ID NO: 20, and SEQ ID NO: 18, respectively. For example, the H-FR1, the H-FR2, the H-FR3, and the H-FR4 of the CD73-targeting antibody or the antigen-binding fragment thereof may comprise the amino acid sequences as set forth in SEQ ID NO: 19, SEQ ID NO: 21, SEQ ID NO: 22, and SEQ ID NO: 18, respectively.

In the present application, the CD73-targeting antibody or the antigen-binding fragment thereof may comprise L-FR1, wherein the C-terminus of the L-FR1 may be linked directly or indirectly to the N-terminus of the LCDR1, and the L-FR1 may comprise the amino acid sequence as set forth in SEQ ID NO: 11 or SEQ ID NO: 23.

In the present application, the CD73-targeting antibody or the antigen-binding fragment thereof may comprise L-FR2, wherein the L-FR2 may be located between the LCDR1 and the LCDR2, and the L-FR2 may comprise the amino acid sequence as set forth in SEQ ID NO: 12 or SEQ ID NO: 24.

In the present application, the CD73-targeting antibody or the antigen-binding fragment thereof may comprise L-FR3, wherein the L-FR3 may be located between the LCDR2 and the LCDR3, and the L-FR3 may comprise the amino acid sequence as set forth in SEQ ID NO: 13, SEQ ID NO: 25, SEQ ID NO: 27, or SEQ ID NO: 28.

In the present application, the CD73-targeting antibody or the antigen-binding fragment thereof may comprise L-FR4, wherein the N-terminus of the L-FR4 may be linked to the C-terminus of the LCDR3, and the L-FR4 may comprise the amino acid sequence as set forth in SEQ ID NO: 14 or SEQ ID NO: 26.

In the present application, the CD73-targeting antibody or the antigen-binding fragment thereof may comprise L-FR1, L-FR2, L-FR3, and L-FR4. For example, the L-FR1, the L-FR2, the L-FR3, and the L-FR4 of the CD73-targeting antibody or the antigen-binding fragment thereof may comprise the amino acid sequences as set forth in SEQ ID NO: 11, SEQ ID NO: 12, SEQ ID NO: 13, and SEQ ID NO: 14, respectively. For example, the L-FR1, the L-FR2, the L-FR3, and the L-FR4 of the CD73-targeting antibody or the antigen-binding fragment thereof may comprise the amino acid sequences as set forth in SEQ ID NO: 23, SEQ ID NO: 24, SEQ ID NO: 25, and SEQ ID NO: 26, respectively. For example, the L-FR1, the L-FR2, the L-FR3, and the L-FR4 of the CD73-targeting antibody or the antigen-binding fragment thereof may comprise the amino acid sequences as set forth in SEQ ID NO: 23, SEQ ID NO: 24, SEQ ID NO: 27, and SEQ ID NO: 26, respectively. For example, the L-FR1, the L-FR2, the L-FR3, and the L-FR4 of the CD73-targeting antibody or the antigen-binding fragment thereof may comprise the amino acid sequences as set forth in SEQ ID NO: 23, SEQ ID NO: 24, SEQ ID NO: 28, and SEQ ID NO: 26, respectively.

In the present application, the CD73-targeting antibody or the antigen-binding fragment thereof may comprise VH, wherein the VH may comprise the amino acid sequence as set forth in SEQ ID NO: 29, SEQ ID NO: 31, SEQ ID NO: 33, or SEQ ID NO: 35.

In the present application, the CD73-targeting antibody or the antigen-binding fragment thereof may comprise VL, wherein the VL may comprise the amino acid sequence as set forth in SEQ ID NO: 30, SEQ ID NO: 32, SEQ ID NO: 34, or SEQ ID NO: 36.

In the present application, the CD73-targeting antibody or the antigen-binding fragment thereof may comprise the VH and the VL. In certain embodiments, the VH may comprise the amino acid sequence as set forth in SEQ ID NO: 29, and the VL may comprise the amino acid sequence as set forth in SEQ ID NO: 30. In certain embodiments, the VH may comprise the amino acid sequence as set forth in SEQ ID NO: 31, and the VL may comprise the amino acid sequence as set forth in SEQ ID NO: 32. In certain embodiments, the VH may comprise the amino acid sequence as set forth in SEQ ID NO: 33, and the VL may comprise the amino acid sequence as set forth in SEQ ID NO: 34. In certain embodiments, the VH may comprise the amino acid sequence as set forth in SEQ ID NO: 35, and the VL may comprise the amino acid sequence as set forth in SEQ ID NO: 36. In certain embodiments, the VH may comprise the amino acid sequence as set forth in SEQ ID NO: 33, and the VL may comprise the amino acid sequence as set forth in SEQ ID NO: 36. In certain embodiments, the VH may comprise the amino acid sequence as set forth in SEQ ID NO: 35, and the VL may comprise the amino acid sequence as set forth in SEQ ID NO: 34.

In the present application, the CD73-targeting antibody or the antigen-binding fragment thereof may comprise at least one CDR in the VH of the present application. In the present application, the CD73-targeting antibody or the antigen-binding fragment thereof may comprise at least one CDR in the VL of the present application. The CDR may be defined according to any definition method.

In the present application, the CD73-targeting antibody or the antigen-binding fragment thereof may comprise the HCDR1, the HCDR2, and the HCDR3 in the VH of the present application. The VH may comprise the amino acid sequence as set forth in SEQ ID NO: 29, SEQ ID NO: 31, SEQ ID NO: 33, or SEQ ID NO: 35.

In the present application, the CD73-targeting antibody or the antigen-binding fragment thereof may comprise the LCDR1, the LCDR2, and the LCDR3 in the VL of the present application. The VL may comprise the amino acid sequence as set forth in SEQ ID NO: 30, SEQ ID NO: 32, SEQ ID NO: 34, or SEQ ID NO: 36.

In the present application, the CD73-targeting antibody or the antigen-binding fragment thereof may comprise an antibody heavy chain constant region. The antibody heavy chain constant region may be derived from a human IgG heavy chain constant region. In certain embodiments, the CD73-targeting antibody or the antigen-binding fragment thereof may comprise an antibody heavy chain constant region, and the antibody heavy chain constant region may be derived from a human IgG1 heavy chain constant region.

In the present application, the CD73-targeting antibody or the antigen-binding fragment thereof may comprise an antibody light chain constant region. The antibody light chain constant region may be derived from a human Igκ constant region.

In the present application, the CD73-targeting antibody or the antigen-binding fragment thereof may comprise an antibody or an antigen-binding fragment thereof.

In certain embodiments, the antigen-binding fragment may include a Fab, a Fab', an Fv fragment, an F(ab')₂, an F(ab)₂, an scFv, a di-scFv, and/or a dAb.

In certain embodiments, the antibody may include a monoclonal antibody, a chimeric antibody, a humanized antibody, and/or a fully human antibody.

For example, the VH of the chimeric antibody may comprise the amino acid sequence as set forth in SEQ ID NO: 29, and the VL of the chimeric antibody may comprise the amino acid sequence as set forth in SEQ ID NO: 30.

For example, the VH of the humanized antibody may comprise the amino acid sequence as set forth in SEQ ID NO: 31, and the VL of the humanized antibody may comprise the amino acid sequence as set forth in SEQ ID NO: 32.

For example, the VH of the humanized antibody may comprise the amino acid sequence as set forth in SEQ ID NO: 33, and the VL of the humanized antibody may comprise the amino acid sequence as set forth in SEQ ID NO: 34.

For example, the VH of the humanized antibody may comprise the amino acid sequence as set forth in SEQ ID NO: 35, and the VL of the humanized antibody may comprise the amino acid sequence as set forth in SEQ ID NO: 36.

For example, the VH of the humanized antibody may comprise the amino acid sequence as set forth in SEQ ID NO: 33, and the VL of the humanized antibody may comprise the amino acid sequence as set forth in SEQ ID NO: 36.

For example, the VH of the humanized antibody may comprise the amino acid sequence as set forth in SEQ ID NO: 35, and the VL of the humanized antibody may comprise the amino acid sequence as set forth in SEQ ID NO: 34.

Furthermore, it should be noted that the CD73-targeting antibody or the antigen-binding fragment thereof of the present application may comprise heavy and/or light chain sequences with one or more conservative sequence modifications thereto. The so-called "conservative sequence modification" refers to an amino acid modification that does not significantly affect or alter the binding properties of the antibody. Such conservative modifications include amino acid substitutions, additions and deletions. Modifications can be introduced into the CD73-targeting antibody or the antigen-binding fragment thereof of the present application by standard techniques known in the art, such as point mutations and PCR-mediated mutations. Conservative amino acid substitutions are those in which an amino acid residue is replaced with an amino acid residue having a similar side chain. Groups of amino acid residues having similar side chains are known in the art. Such groups of amino acid residues include amino acids with basic side chains (e.g., lysine, arginine, and histidine), amino acids with acidic side chains (e.g., aspartic acid, and glutamic acid), amino acids with uncharged polar side chains (e.g., glycine, asparagine, glutamine, serine, threonine, tyrosine, cysteine, and tryptophan), amino acids with non-polar side chains (e.g., alanine, valine, leucine, isoleucine, proline, phenylalanine, and methionine), amino acids with β-branched side chains (e.g., threonine, valine, and isoleucine), and amino acids with aromatic side chains (e.g., tyrosine, phenylalanine, tryptophan, and histidine). In certain embodiments, one or more amino acid residues in the CDRs of the CD73-targeting antibody or the antigen-binding fragment thereof of the present application can be replaced with other amino acid residues of the same side chain group. Those skilled in the art know that some conservative sequence modifications do not eliminate antigen binding, as can be seen, for example, in Brummell et al., (1993) Biochem 32: 1180-8; de Wildt et al., (1997) Prot. Eng. 10: 835-41; Komissarov et al., (1997) J. Biol. Chem. 272: 26864-26870; Hall et al., (1992) J. Immunol. 149: 1605-12; Kelley and O'Connell (1993) Biochem. 32: 6862-35; Adib-Conquy et al., (1998) Int. Immunol. 10: 341-6 and Beers et al., (2000) Clin. Can. Res. 6: 2835-43.

The CD73 antigen-binding protein of the present application can be identified, screened, or characterized by various assays known in the art.

For example, the antigen-binding activity of the antigen-binding protein or the fusion protein of the present application can be tested, for example, by known methods such as enzyme-linked immunosorbent assay (ELISA), immunoblotting (e.g., western blotting), flow cytometry (e.g., FACS), immunohistochemistry, and immunofluorescence.

In the present application, the CD73-targeting antibody or the antigen-binding fragment thereof is capable of specifically binding to CD73 or a functionally active fragment thereof.

In certain embodiments, the CD73-targeting antibody or the antigen-binding fragment thereof can be screened for affinity by Biacore. The CD73-targeting antibody or the antigen-binding fragment thereof may bind to a CD73 protein with a KD of 5 × 10⁻⁹ M or less; for example, the antigen-binding protein of the present application can bind to CD73 with a KD of less than about 4 × 10⁻⁹ M, less than about 3 × 10⁻⁹ M, less than about 2 × 10⁻⁹ M, less than about 1 × 10⁻⁹ M, less than about 7 × 10⁻¹⁰ M, less than about 6 × 10⁻¹⁰ M, less than about 5 × 10⁻¹⁰ M, less than about 4 × 10⁻¹⁰ M, less than about 3 × 10⁻¹⁰ M, less than about 2 × 10⁻¹⁰ M, less than about 1 × 10⁻¹⁰ M, less than about 9 × 10⁻¹¹ M, less than about 5 × 10⁻¹¹ M, or less than about 2 × 10⁻¹¹ M. In certain embodiments, the present application also includes the use of the FACS method to determine the binding activity of the antigen-binding protein to an antigen. For example, the antigen-binding protein of the present application may bind to human CD73 on the cell surface with an EC50 value of less than or equal to about 0.5 µg/mL, less than or equal to about 0.4 µg/mL, less than or equal to about 0.3 µg/mL, less than or equal to about 0.2 µg/mL, or less than or equal to about 0.1 µg/mL. For example, the antigen-binding protein of the present application may bind to human CD73 on the cell surface with an EC50 value of less than or equal to about 0.4 µg/mL, less than or equal to about 0.3 µg/mL, less than or equal to about 0.2 µg/mL, or less than or equal to about 0.1 µg/mL.

In certain embodiments, the CD73-targeting antibody or the antigen-binding fragment thereof is capable of binding to CD73. In certain cases, the antigen-binding protein of the present application can also cross-react with monkey CD73, for example, as detected by FACS. In the present application, "cross-reactivity" generally refers to the ability of an antibody to react with homologous proteins from other species.

In certain embodiments, the CD73-targeting antibody or the antigen-binding fragment thereof of the present application does not bind to mouse and/or rat CD73.

For example, the titer can be detected by the ELISA method. For example, the binding activity or functional activity of the CD73-targeting antibody or the antigen-binding fragment thereof can be detected using FACS detection. For example, the CD73-targeting antibody or the antigen-binding fragment thereof can be subjected to an affinity assay.

In the present application, the CD73-targeting antibody or the antigen-binding fragment thereof is capable of preventing or reducing activation of CD73. For example, the CD73-targeting antibody or the antigen-binding fragment thereof is capable of preventing or reducing the conversion of CD73 from a catalytically inactive open conformation to a catalytically active closed conformation. For example, the CD73-targeting antibody or the antigen-binding fragment thereof is capable of closing the catalytic center of CD73, thereby inhibiting the enzymatic activity of CD73.

In certain embodiments, the binding epitope of the CD73-targeting antibody or the antigen-binding fragment thereof can be determined by hydrogen-deuterium exchange mass spectrometry (HDX-MS). In certain embodiments, the CD73-targeting antibody or the antigen-binding fragment thereof has activity in competing with a CPI-006 antibody (Corvus) for binding.

In the present application, when binding to the CD73, the CD73-targeting antibody or the antigen-binding fragment thereof binds to at least one amino acid residue in the 143-NIKAKGPLASQISGL-157 region (SEQ ID NO: 37) at the N-terminal domain of CD73 or its corresponding CD73 sequence, the 178-SKETPFLSNPGTNL-191 region (SEQ ID NO: 38) or its corresponding CD73 sequence, and the 381-WNHVSM-386 region (SEQ ID NO: 39) at the C-terminal domain of CD73 or its corresponding CD73 sequence.

In the present application, "its corresponding CD73 sequence" may refer to an amino acid sequence at a corresponding position in a homolog of CD73 (or CD73 from other species).

In the present application, the CD73-targeting antibody or the antigen-binding fragment thereof is capable of inhibiting the enzymatic activity of CD73. For example, the CD73-targeting antibody or the antigen-binding fragment thereof may have an inhibitory effect on the enzymatic activity of recombinant CD73. For example, the CD73-targeting antibody or the antigen-binding fragment thereof may have an inhibitory effect on the enzymatic activity of human CD73. The enzymatic activity of the present application can be detected by any method commonly used in the art. The detection of the enzymatic activity may include mixing the CD73 antigen-binding protein with a recombinant CD73 protein, adding AMP and ATP, then adding a CellTiter-Glo^{®} substrate into a CellTiter-Glo^{®} buffer, mixing uniformly and equilibrating to room temperature for reaction, and performing full-wavelength detection. For example, the CD73-targeting antibody or the antigen-binding fragment thereof may inhibit the enzymatic activity of the recombinant CD73 protein with an EC50 value of less than or equal to about 0.8 µg/mL, less than or equal to about 0.7 µg/mL, less than or equal to about 0.6 µg/mL, less than or equal to about 0.5 µg/mL, less than or equal to about 0.4 µg/mL, less than or equal to about 0.3 µg/mL, or less than or equal to about 0.2 µg/mL. For example, the CD73-targeting antibody or the antigen-binding fragment thereof may inhibit the enzymatic activity of a CD73 protein on the cell membrane with an EC50 value of less than or equal to about 6 µg/mL, less than or equal to about 5 µg/mL, less than or equal to about 4 µg/mL, less than or equal to about 3 µg/mL, less than or equal to about 2 µg/mL, less than or equal to about 1 µg/mL, or less than or equal to about 0.5 µg/mL.

In the present application, the CD73-targeting antibody or the antigen-binding fragment thereof is capable of inducing endocytosis of CD73 on a cell surface. In certain embodiments, the endocytosis of the protein can be assessed by a fluorescence labeling method. For example, the efficiency of the antigen-binding protein in inducing endocytosis of CD73 on the cell surface can be obtained by detecting the fluorescence intensity of cells using flow cytometry. In certain cases, the CD73-targeting antibody or the antigen-binding fragment thereof may induce endocytosis of CD73 with an efficiency of about 50% or higher.

### Antibody-drug conjugate and compound

In another aspect, the present application provides an antibody-drug conjugate.

In the present application, the antibody-drug conjugate comprises the CD73-targeting antibody or the antigen-binding fragment thereof of the present application.

In the present application, the antibody-drug conjugate may comprise a structure represented by formula 1: M-(L1)ₐ-(L2)_{b}-D (formula 1), wherein M represents the CD73-targeting antibody or the antigen-binding fragment thereof of the present application; L1 represents a linker linking to M, and L2 represents a linker linking to D; a and b are each independently selected from 0-10; D represents a drug.

In the present application, provided is a compound for preparing the antibody-drug conjugate of the present application, which has a structure represented by formula 2: M-(L1)ₐ (formula 2), wherein M represents the CD73-targeting antibody or the antigen-binding fragment thereof of the present application; L1 represents a linker linking to M; a is selected from 0-10.

### Modification of M and linkage to L1

In the present application, the L1 may be linked to the M by a sulfhydryl group, an azido group, or an amide group on the M.

In the present application, the M may be an intact antibody structure (e.g., an intact IgG antibody structure comprising 2 identical light chains and 2 identical heavy chains). In the present application, the light chain constant region, the CH1 of the heavy chain constant region, and/or the hinge region of the M may comprise linking sites capable of linking to the L1. For example, the light chain constant region, the CH1 of the heavy chain constant region, and the hinge region of the M may comprise linking sites capable of linking to the L1.

In the present application, the linking site may comprise a cysteine. In the present application, the linking site may have a sulfhydryl group in a linkable state. In the present application, the number of the linking sites may be 8. In the present application, the linking sites may include: 2 linking sites located in the light chain constant region, 2 linking sites located in the CH1 of the heavy chain constant region, and 4 linking sites located in the hinge region.

In the present application, the linking site may comprise a group capable of linking to the L1 following deglycosylation modification. In the present application, the linking site may comprise a group capable of linking to the L1 following glycosylation modification.

Glycosylation of a protein can depend on the amino acid sequence of the protein (e.g., the CD73-targeting antibody or the antigen-binding fragment thereof of the present application) and the host cell in which the protein is expressed. Different organisms may produce different glycosylation enzymes (e.g., glycosyltransferases and glycosidases), and have different available substrates (nucleotide sugars). Due to these factors, the protein glycosylation pattern and the composition of the residue of the glycosylation modification site may vary depending on the host system in which the specific protein is expressed. Glycosyl residues that can be used in the present application may include glucose, galactose, mannose, fucose, N-acetylglucosamine, and/or sialic acid. For example, the glycosylation modification may include patterns of glycosylation modifications adapted to humans.

In the present application, different protein glycosylations can affect and/or result in different protein properties (e.g., changes in properties, such as expression level, *in vivo* half-life, protein folding, solubility, susceptibility to proteases, trafficking, transport, compartmentalization, secretion, recognition by other proteins or factors, antigenicity, allergenicity, or the like).

### Linker

In the present application, the a may be an integer selected from 0-10, for example, 0, 1, 2, 3, 4, 5, 6, 7, 8, 9, or 10.

In the present application, the b may be an integer selected from 0-10, for example, 0, 1, 2, 3, 4, 5, 6, 7, 8, 9, or 10.

In the present application, the L1 and/or the L2 may be selected from the group consisting of: a cleavable linker, a non-cleavable linker, a hydrophilic linker, a hydrophobic linker, a charged linker, an uncharged linker, and a dicarboxylic acid-based linker.

In the present application, both the L1 and the L2 may be considered to belong to linkers.

In the present application, the linker may be a moiety that stretches the drug linkage to avoid, for example, shielding the active site of the antibody or to improve the solubility of the ADC. The linker may comprise a stretcher unit and/or an amino acid unit.

In the present application, the linker may be used to conjugate (e.g., covalently link) an antibody (e.g., the CD73-targeting antibody or the antigen-binding fragment thereof of the present application) to the drug.

For example, the conjugation may be achieved by a cysteine sulfhydryl group or an amine (e.g., N-terminus or amino acid side chain such as lysine) of the antibody forming a bond with a functional group of the linker.

For example, the linker may have a functional group capable of reacting with a free cysteine present in the antibody to form a bond (e.g., a covalent bond). For example, the functional group may include: maleimide, haloacetamides, α-haloacetyl, active esters such as succinimide esters, 4-nitrophenyl esters, pentafluorophenyl esters, tetrafluorophenyl esters, anhydrides, acid chlorides, sulfonyl chlorides, isocyanates, and/or isothiocyanates.

In some cases, the linker may comprise a functional group capable of reacting with an electrophilic group present in the antibody. For example, the functional group may include: aldehyde, ketone carbonyl, hydrazine, oxime, amino, hydrazine, thiosemicarbazone, hydrazine carboxylate, and/or arylhydrazide.

In the present application, the linker may include cleavable linkers and non-cleavable linkers. For example, the linker (e.g., L2) may be a cleavable (e.g., self immolative) linker, which may facilitate the release of the drug. For example, the linker (e.g., L1) may be a non-cleavable linker, which may link to the antibody and is non-cleavable. In the present application, the cleavable linker may be cleaved under intracellular conditions. For example, the cleavable linker may include: acid-labile linkers (e.g., comprising hydrazone), protease-sensitive (e.g., peptidase-sensitive) linkers, photolabile linkers, and/or disulfide-containing linkers. For example, the cleavable linker may include peptide linkers capable of being cleaved by intracellular proteases (e.g., lysosomal proteases) and/or endosomal proteases. In certain cases, the linker is cleavable under intracellular conditions. For example, cleavage of the linker may allow the drug in the antibody-drug conjugate to exert an effective therapeutic effect.

In the present application, the structure and/or properties of the linker are stable outside cells. The antibody-drug conjugate of the present application may be structurally stable before transport or delivery into a cell. For example, in the antibody-drug conjugate, the CD73-targeting antibody or the antigen-binding fragment thereof remains conjugated to the drug.

In certain cases, if the linker in the antibody-drug conjugate is capable of, for example, maintaining the specific binding properties of the CD73-targeting antibody or the antigen-binding fragment thereof, it participates in the delivery of the antibody-drug conjugate, and/or maintains the therapeutic effect (e.g., cytotoxic effect) of the drug.

In the present application, the linker may include hydrophilic linkers (e.g., PEG4Mal and sulfo-SPDB) and hydrophobic linkers. For example, the hydrophilic linker can reduce the extent to which the antibody-drug conjugate may be pumped out of resistant cancer cells through MDR (multiple drug resistance) or functionally similar transporters.

In the present application, the linker may also function to inhibit cell growth and/or cell proliferation, either directly or indirectly. For example, the linker may function as an intercalator in the cleavage. For example, the linker may inhibit macromolecular biosynthesis.

In the present application, the linker may facilitate entry of the antibody-drug conjugate into a cell (e.g., may facilitate an "internalization" effect). In the present application, the linker may also be designed to improve the stability of the antibody-drug conjugate.

In the present application, the L1 may react with the sulfhydryl group to achieve linkage.

In the present application, the L1 may be selected from the group consisting of: 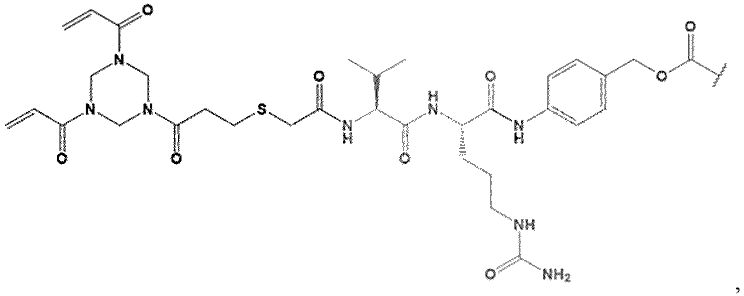 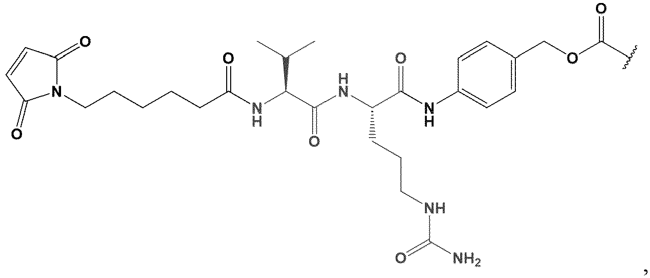 and

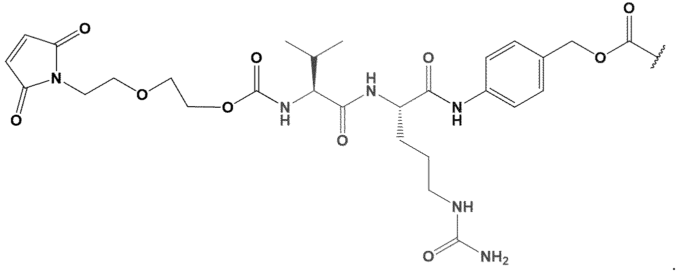

In the present application, the L2 may be selected from the group consisting of: a polypeptide, N-succinimidyl-3-(2-pyridyldithio)propionate (SPDP), N-succinimidyl 4-(2-pyridyldithio)pentanoate (SPP), N-succinimidyl 4-(2-pyridyldithio)butyrate (SPDB), N-succinimidyl-4-(2-pyridyldithio)-2-sulfobutyrate (sulfo-SPDB), N-succinimidyl iodoacetate (SIA), N-succinimidyl (4-iodoacetyl)aminobenzoate (SIAB), maleimide PEG NHS, succinimidyl 4-(N-maleimidomethyl)cyclohexane-1-carboxylate (SMCC), sulfosuccinimidyl 4-(N-maleimidomethyl)cyclohexane-1-carboxylate (sulfo-SMCC), and 2,5-dioxopyrrolidin-1-yl 17-(2,5-dioxo-2,5-dihydro-1H-pyrrol-1-yl)-5,8,11,14-tetraoxo-4,7,10,13-tetraazaoctadecan-1-oate (CX1-1). In the present application, the L2 may be GGFG, LP, VK, VC, GFG, GGFGG, GGFGS, GGFGGG, GGFGGE, GGFGGGFG, DGGF, DGGFG, D^{d} GGFG, DG^{Me} GFG, DGGFS, DDGGFG, KDGGFG, KGGFG, EGGFG, or SGGFG. For example, the L2 may be GGFG. The cathepsin B-cleavable tetrapeptide Gly-Gly-Phe-Gly (GGFG) is a highly hydrophilic linker (e.g., more hydrophilic than Gly-Phe-Leu-Gly).

In the present application, the L2 may be

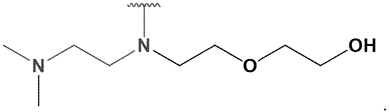

In the present application, the L1-L2 may be selected from the group consisting of: 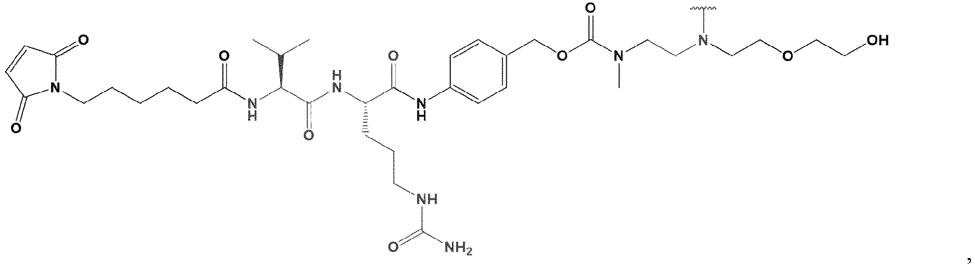 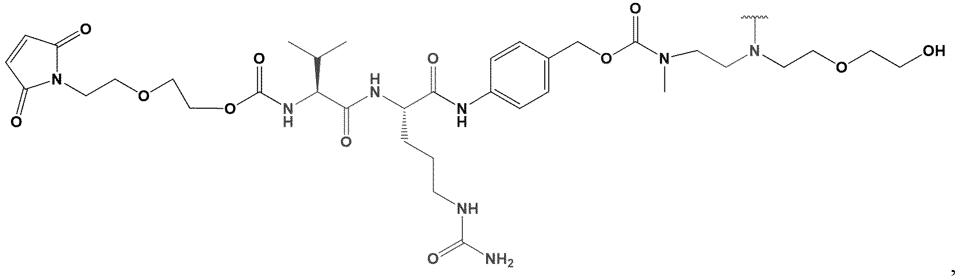 and

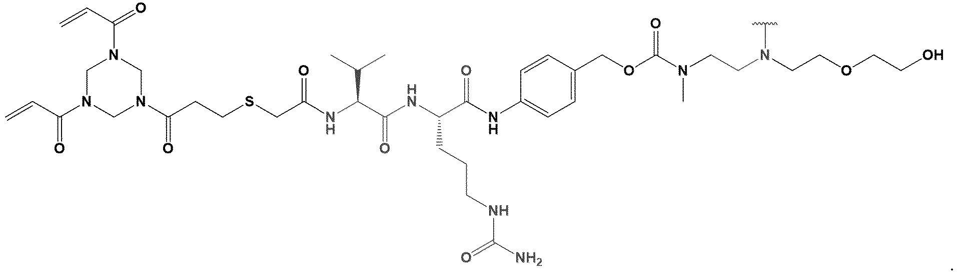

### Drug

In the present application, the drug may have an ability to kill tumor cells, and/or an ability to inhibit tumor cell growth.

In the present application, the drug may include a small-molecule drug.

In the present application, the drug may be selected from the group consisting of: a V-ATPase inhibitor, a Bcl2 inhibitor, an MCL1 inhibitor, an HSP90 inhibitor, an IAP inhibitor, an mTor inhibitor, a microtubule stabilizer, a microtubule destabilizer, auristatin, dolastatin, a maytansinoid, MetAP (methionine aminopeptidase), an inhibitor of nuclear export of protein CRM1, a DPPIV inhibitor, a proteasome inhibitor, an inhibitor of phosphoryl transfer reactions in mitochondria, a protein synthesis inhibitor, a CDK2 inhibitor, a CDK9 inhibitor, a kinesin inhibitor, an HDAC inhibitor, a DNA damaging agent, a DNA alkylating agent, a DNA intercalator, a DNA minor groove binder, a DHFR inhibitor, a nucleoside analog, an HD AC inhibitor, an anthracycline, a NAMPT inhibitor, SN-38 glucuronic acid, etoposide phosphate, nitrogen mustard, a proteasome inhibitor, a cytokine, a tubulysin B analog, and a Toll-like receptor agonist.

In the present application, the drug may be selected from the group consisting of: DM1, exatecan, Dxd, MMAE, calicheamicin, anthracyclin-5G, DM4, microtubule inhibitor SHR153024, PNU-159682, eribulin, Tub 196, Duo5 toxin, an SN38 derivative, or derivatives thereof.

In the present application, the drug may be a DNA topoisomerase I inhibitor. In the present application, the drug may be a camptothecin derivative.

In the present application, tubulysin B may be a cytotoxic active tubulin that is capable of inhibiting tubulin polymerization and leading to cell cycle arrest and apoptosis. Tubulysin B has a CAS number of 205304-87-6. In the present application, MMAE (monomethyl auristatin E) is a synthetic derivative of dolastatin 10, which can effectively inhibit mitosis by inhibiting tubulin polymerization. MMAE has a CAS number of 474645-27-7. In the present application, SN38 is an active metabolite of the topoisomerase I inhibitor irinotecan. It has a CAS number of 119577-28-5. In the present application, the drug may be SN38 or MMAE.

For example, the drug may have the following structure:

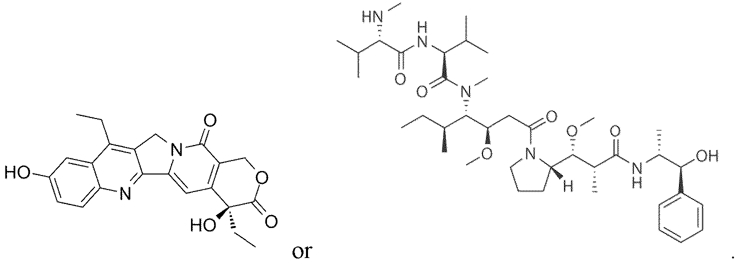

In the present application, the (L1)ₐ-(L2)_{b}-D may have a structure selected from the group consisting of: 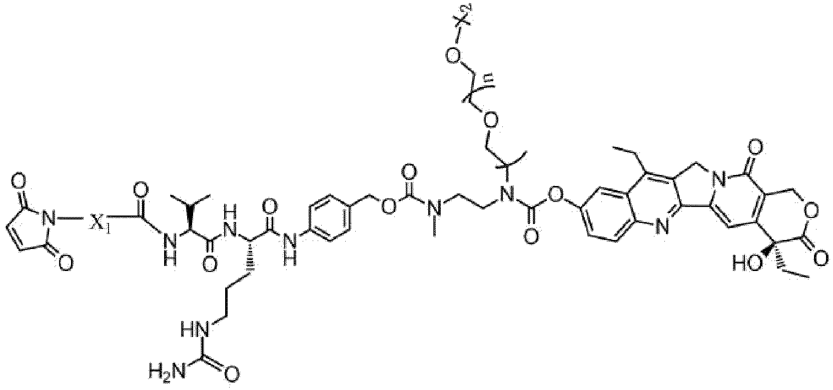 wherein
X₁ is an alkane chain or a PEG chain, and
X₂ is H or -C(O)NR¹R², and
R¹ is selected from hydrogen, halogen, hydroxyl, substituted or unsubstituted C₁₋₆ alkyl, substituted or unsubstituted C₁₋₆ hydroxyalkyl, substituted or unsubstituted C₁₋₆ aminoalkyl, substituted or unsubstituted C₁₋₆ alkoxy, substituted or unsubstituted C₁₋₆ alkanoyl, and a substituted or unsubstituted C₁₋₆ alkyl aldehyde group;
R² is selected from hydrogen and substituted or unsubstituted C₁₋₆ alkyl; each substituted C₁₋₆ alkyl is optionally substituted with 1-5 substituents independently selected from: halogen, hydroxyl, amino, carbonyl, carboxyl, 5- to 10-membered heteroaryl, and C₁₋₆ haloalkyl, wherein the 5- to 10-membered heteroaryl has 1-3 heteroatoms selected from oxygen, nitrogen, and sulfur; n is an integer greater than or equal to 1 and less than or equal to 20.

In the present application, the (L1)ₐ-(L2)_{b}-D may have a structure selected from the group consisting of: 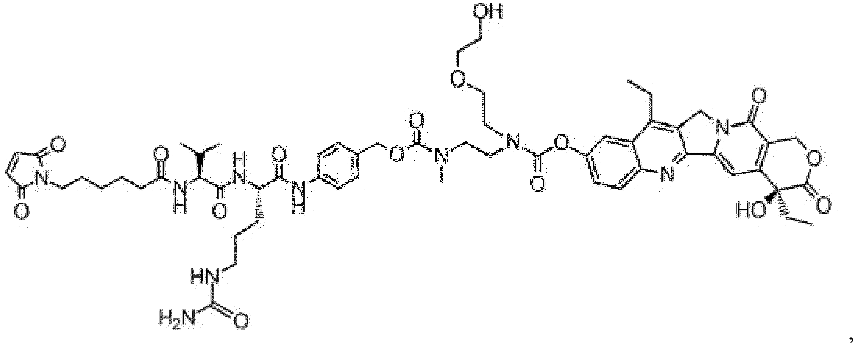 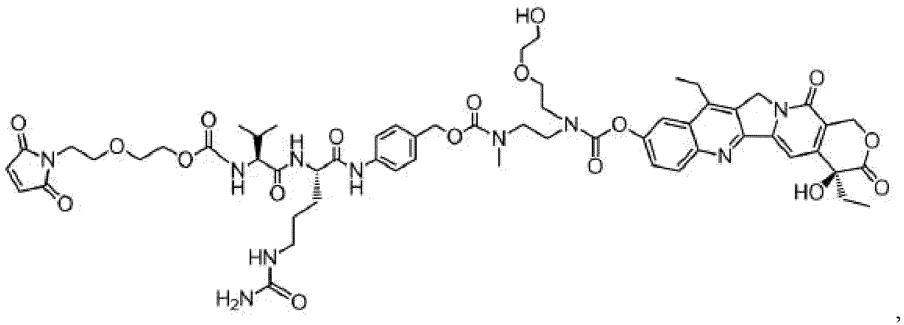 and

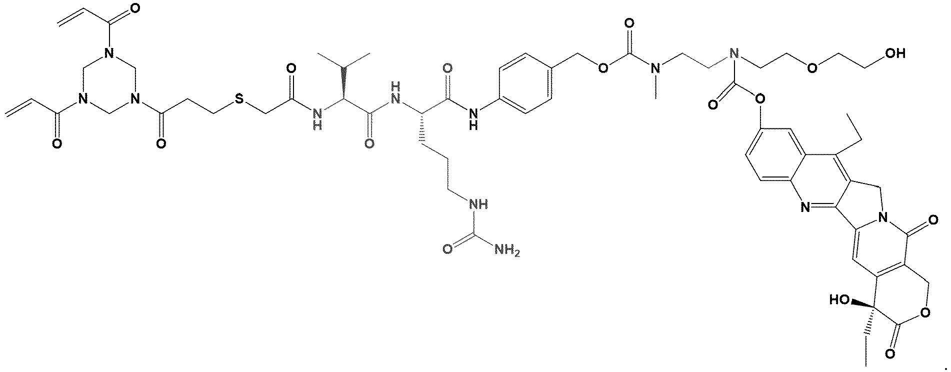

In the present application, the antibody-drug conjugate may have a structure selected from the group consisting of: 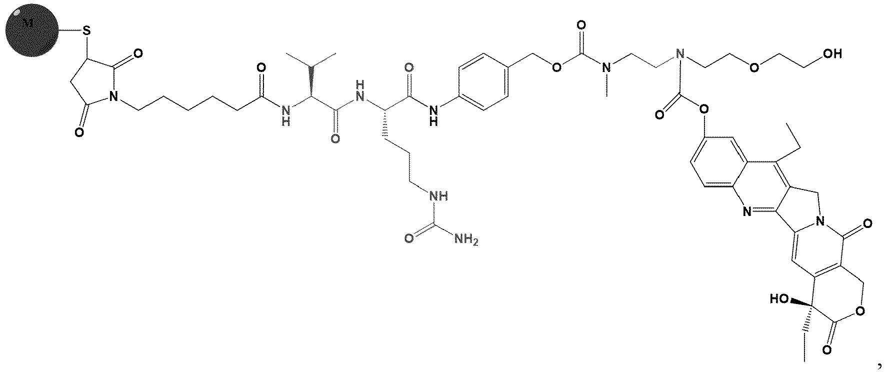 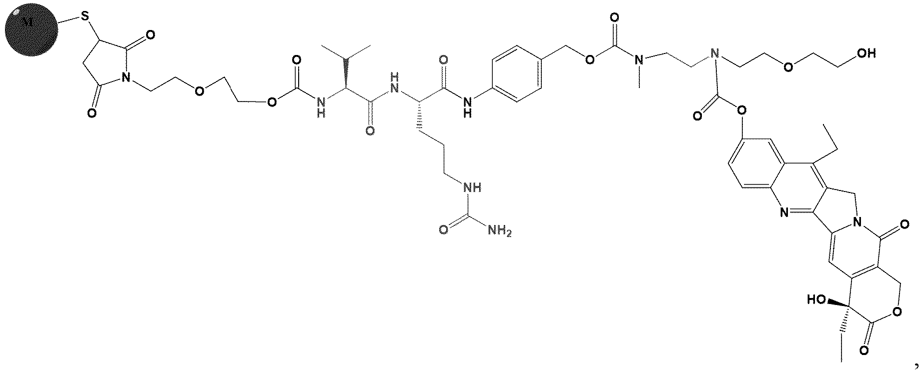 and

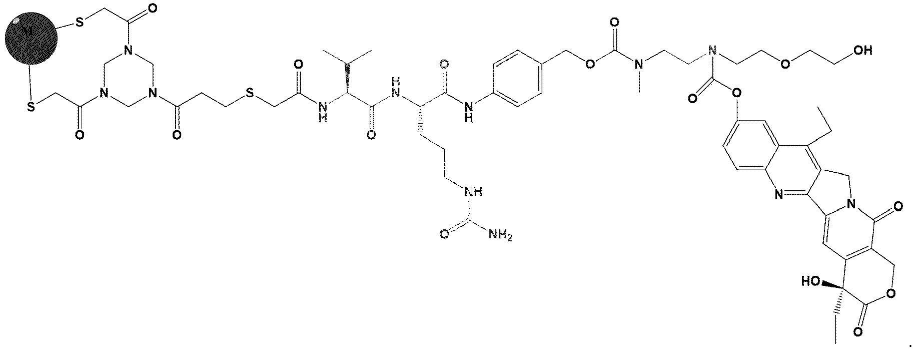

In the present application, the antibody-drug conjugate may have a structure selected from the group consisting of: 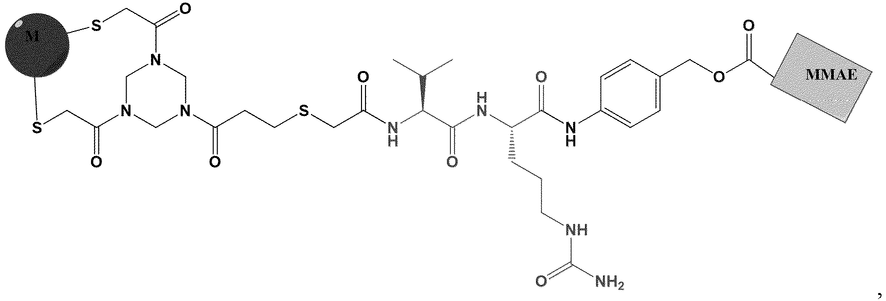 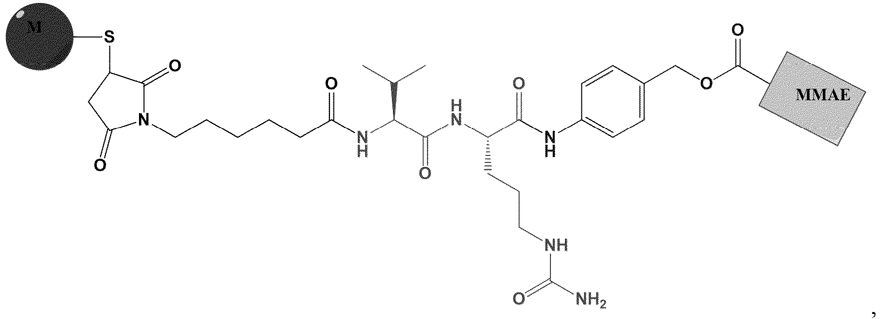 and

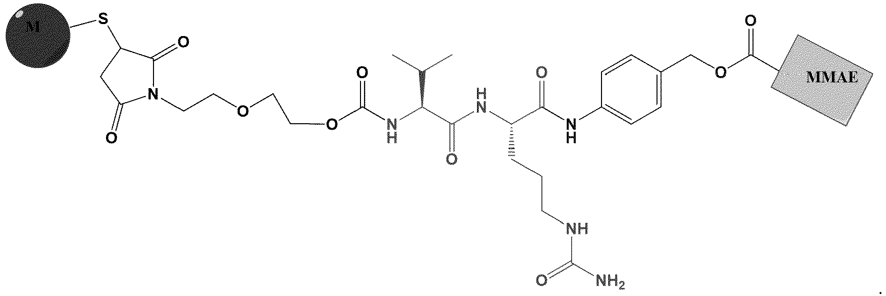

In the present application, the antibody-drug conjugate may have a drug-to-antibody ratio of about 1-8. For example, the drug-to-antibody ratio may be about 1, about 2, about 3, about 4, about 5, about 6, about 7, or about 8. For example, the drug-to-antibody ratio may be about 8. For example, the drug-to-antibody ratio may be about 7.8.

### Compound and preparation method

In another aspect, the present application provides a compound for the antibody-drug conjugate of the present application, which has a structure represented by formula 2: M-(L1)ₐ (formula 2), wherein M represents the CD73-targeting antibody or the antigen-binding fragment thereof of the present application; L1 represents a linker linking to M; a is selected from 0-10.

In the present application, the compound may comprise a structure selected from the group consisting of: 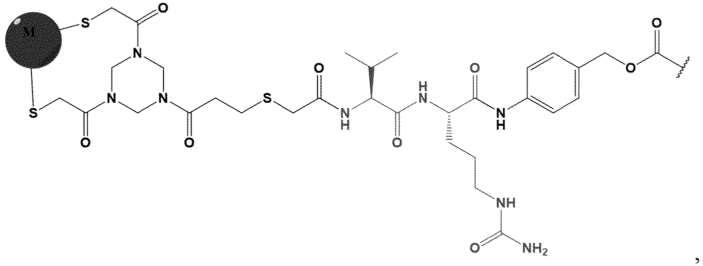 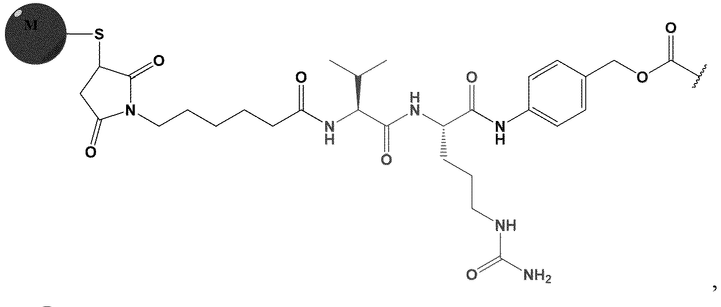 and

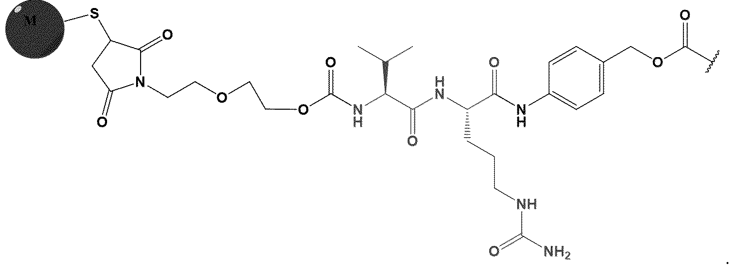

In another aspect, the present application provides a method for preparing the antibody-drug conjugate of the present application, comprising the following step: contacting the compound of the present application with the drug of the present application.

In some cases, the method for preparing the antibody-drug conjugate may comprise the following steps: (1) contacting the L1 and the L2 of the present application with the drug; (2) obtaining a compound (L1)ₐ-(L2)_{b}-D; and (3) contacting the M of the present application with the compound (L1)ₐ-(L2)_{b}-D to obtain the antibody-drug conjugate of the present application. For example, the M in step (3) is subjected to the glycosylation modification.

### Pharmaceutical composition and use

In another aspect, the present application provides a pharmaceutical composition, comprising the antibody-drug conjugate of the present application or a pharmaceutically acceptable carrier.

In the present application, the pharmaceutical composition may comprise a "therapeutically effective dose" or "prophylactically effective dose" of the antibody-drug conjugate of the present application. The "therapeutically effective dose" may refer to an amount that is effective to achieve the desired therapeutic result, given the necessary dose and duration. The therapeutically effective dose can be determined by those skilled in the art and, for example, may vary depending on factors such as the state of the disease, the age, sex, and body weight of the subject, and the ability of the antibody-drug conjugate to elicit a desired response in the subject. The "prophylactically effective dose" may refer to an amount that is effective to achieve the desired prophylactic result, given the necessary dose and duration. For example, the prophylactically effective dose may be lower than the therapeutically effective dose.

In the present application, the pharmaceutical composition may be formulated in a form suitable for administration (e.g., suitable for parenteral, intradermal, intramuscular, intraperitoneal, intravenous and subcutaneous, intratumoral and/or mucosal administration). In the present application, the pharmaceutical composition may comprise other pharmaceutically active ingredients.

In the present application, the pharmaceutically acceptable carrier may include any and all solvents, dispersion media, coatings, antibacterial and antifungal agents, isotonic agents and absorption delaying agents, and the like, that are physiologically compatible. The pharmaceutically acceptable carrier may include one or more of water, saline, phosphate-buffered saline, dextrose, glycerol, ethanol, and the like, as well as combinations thereof. The pharmaceutically acceptable carrier may also include isotonic agents, wetting agents, emulsifying agents, preservatives, and/or buffering agents.

In another aspect, the present application provides a method for modulating a tumor microenvironment in a subject, comprising the following step: administering to the subject the antibody-drug conjugate of the present application or the pharmaceutical composition of the present application.

In another aspect, the present application provides a method for modulating an immune response in a subject, comprising the following step: administering to the subject the antibody-drug conjugate of the present application or the pharmaceutical composition of the present application.

The present application provides use of the antibody-drug conjugate of the present application or the pharmaceutical composition of the present application in the preparation of a medicament, wherein the medicament can modulate a tumor microenvironment in a subject and/or modulate an immune response in a subject.

The present application provides the antibody-drug conjugate, or the pharmaceutical composition of the present application for use in modulating a tumor microenvironment in a subject and/or modulating an immune response in a subject.

In another aspect, the present application provides use of the antibody-drug conjugate of the present application or the pharmaceutical composition of the present application in the preparation of a medicament, wherein the medicament is capable of preventing and/or treating a tumor.

The present application provides a method for preventing and/or treating a tumor, comprising the following step: administering to a subject a medicament prepared with the antibody-drug conjugate of the present application or the pharmaceutical composition of the present application.

The present application provides the antibody-drug conjugate or the pharmaceutical composition of the present application for use in preventing and/or treating a tumor.

In the present application, the tumor may include a solid tumor and/or a non-solid tumor.

For example, the tumor may include breast cancer (e.g., triple-negative breast cancer) and/or pancreatic cancer.

Without being bound by any theory, the following examples are intended only to illustrate various technical solutions of the invention in the present application, but not to limit the scope of the invention in the present application.

### Examples

The chemical formula of the SN-38 small molecule is as follows: 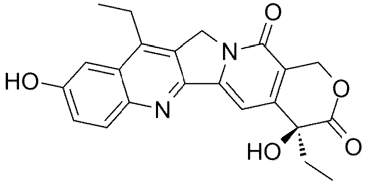
900698 antibody: having a heavy chain variable region VH with the amino acid sequence as set forth in SEQ ID NO: 35, and a light chain variable region VL with the amino acid sequence as set forth in SEQ ID NO: 34;
900725 antibody: having a heavy chain variable region VH with the amino acid sequence as set forth in SEQ ID NO: 53, and a light chain variable region VL with the amino acid sequence as set forth in SEQ ID NO: 54;

### Example 1. Preparation of Antibody-Drug Conjugates

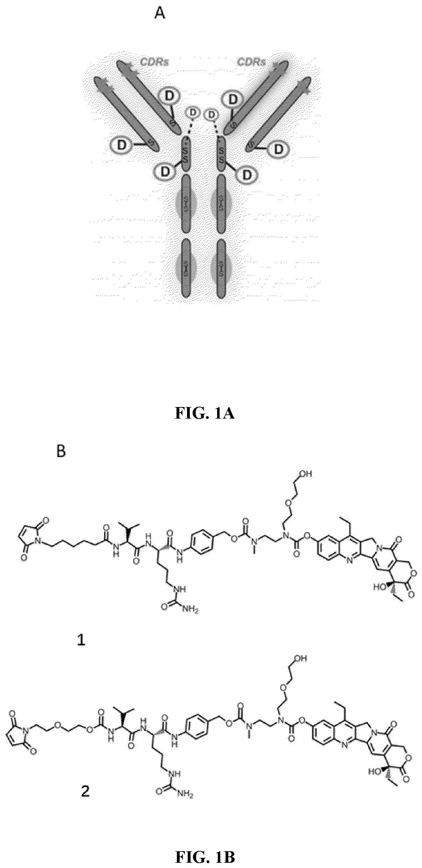

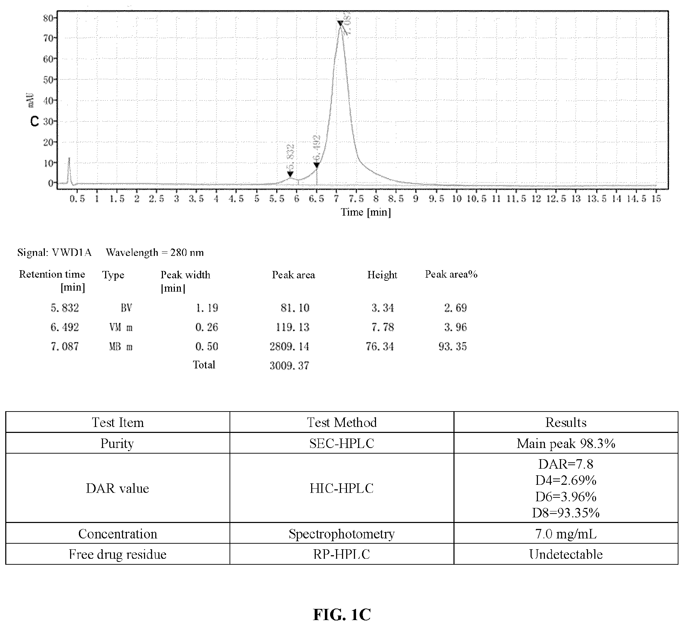

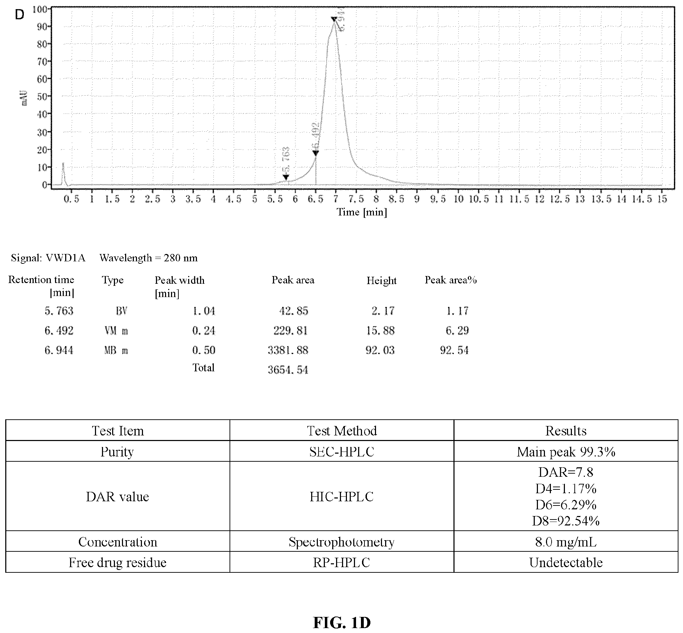

SN-38 small molecules were conjugated to 900698 antibodies using cysteine conjugation, and the drug-to-antibody ratios (DAR values) of the ADC molecules were determined using hydrophobic chromatography. The corresponding structures and DAR values are shown in FIGs. 1A-1D below. FIG. 1A shows a structural schematic diagram of CD73-ADC, where the antibody molecule was derived from 900698, and D represents the small-molecule drug SN-38; FIG. 1B shows the molecular structures of two different linker-SN38 molecules; FIG. 1C shows the HIC detection results and the DAR value of the CD73 antibody-Mc-VC-PAB-DMEDA-PEG-SN38 molecule (i.e., antibody-drug conjugate 1 formed by linkage of 900698-molecule 1 of FIG. 1B); FIG. 1D shows the HIC detection results and the DAR value of the CD73 antibody-MP2-VC-PAB-DMEDA-PEG-SN38 molecule (i.e., antibody-drug conjugate 2 formed by linkage of 900698-molecule 2 of FIG. 1B).

The results in FIG. 1 show that the DAR values of the CD73 antibody-Mc-VC-PAB-DMEDA-PEG-SN38 (abbreviated as antibody-drug conjugate 1) and CD73 antibody-MP2-VC-PAB-DMEDA-PEG-SN38 (abbreviated as antibody-drug conjugate 2) molecules were both about 7.8.

### Example 2. Assay for Cell Binding Activity of Antibody-Drug Conjugates to Human CD73 Antigen Expressed on Membrane

The binding activity of the antibody-drug conjugate 1 and the antibody-drug conjugate 2 prepared in Example 1 and the 900698 naked antibody to cells expressing human CD73 (CHOK1-huCD73-3F4, Huabo Biopharm) was assayed.

All antigen-binding proteins were diluted to 30 µg/mL with a solution of 1% BSA in PBS (1% BSA/PBS), followed by a 3-fold dilution across 10 gradients. The diluted proteins were each added at 20 µL per well to a 96-well U-bottom plate, with a negative control (adding only 1% BSA/PBS) set up simultaneously. A suspension of cells expressing human CD73 (CHOK1-huCD73-3F4, Huabo Biopharm) in the logarithmic growth phase was taken and centrifuged (1000 rpm × 5 min), and then the culture medium was discarded. The cells were resuspended in 1% BSA/PBS until the viable cell density was 1 × 10⁶ cells/mL, then added at 20 µL (2 × 10⁴ cells) per well to the 96-well U-bottom plate containing the anti-CD73 antigen-binding proteins, and incubated at room temperature for reaction for 30 min. The reacted cells in the 96-well U-bottom plate were resuspended in 1% BSA/PBS and centrifuged (300 g × 3 min), and then the supernatant was discarded. This washing process was performed once. Then, a 1:200 dilution of PE-goat anti-human-Fc (Jackson ImmunoResearch, #109-115-098) was added, and the cells were incubated in the dark at room temperature for reaction for 15 min. The reacted cells in the 96-well U-bottom plate were further resuspended in 1% BSA/PBS and centrifuged (300 g × 3 min), and then the supernatant was discarded. This washing process was performed 3 times. Finally, the cells were resuspended in 100 µL of 1% BSA/PBS per well, and the fluorescence intensity in the PE channel was detected using a flow cytometer (BD, #Canto II).

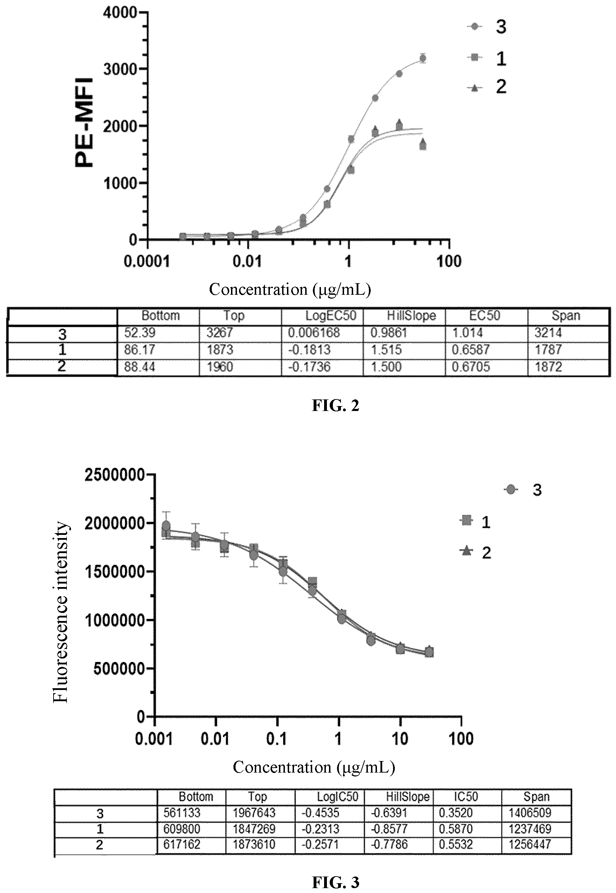

The results for the binding activity of the antibody-drug conjugate 1, the antibody-drug conjugate 2, and the 900698 naked antibody are shown in FIG. 2 (represented as 1-3 in FIG. 2, respectively). The results show that all three proteins had a good affinity for the cells expressing human CD73 (CHOK1-huCD73-3F4, Huabo Biopharm); the antibody-drug conjugate 1, the antibody-drug conjugate 2, and the 900698 naked antibody had a comparable affinity for the cells expressing human CD73 (CHOK1-huCD73-3F4, Huabo Biopharm), indicating that the affinity of the 900698 naked antibody for the CD73 antigen on the cell surface was not affected after conjugation to the SN38 small-molecule toxin.

### Example 3. Assay for Inhibitory Effects of Antibody-Drug Conjugates on Enzymatic Activity of Soluble Recombinant CD73 Antigen Protein

The inhibitory effects of the antibody-drug conjugate 1 and the antibody-drug conjugate 2 prepared in Example 1 and the 900698 naked antibody on the enzymatic activity of a recombinant CD73 protein (ACRO, #CD3-H52H7-100 µg) were assayed.

The specific procedures were as follows: The series of CD73 antigen-binding proteins were diluted to 120 µg/mL with TM Buffer (ROCKLAND, #MB-059), followed by a 3-fold dilution across 10 gradients. The diluted proteins were each added at 25 µL/well to a 96-well plate. The recombinant CD73 protein was then diluted to 0.3 µg/mL with TM Buffer (ROCKLAND, #MB-059) and added at 25 µL/well to the 96-well plate. The mixture was mixed uniformly. Then, diluted AMP and ATP were mixed at a ratio of 1:1, and the mixture was added at 50 µL/well to the above 96-well plate. The resulting mixture was allowed to react for 15 min. Finally, a CellTiter-Glo^{®} substrate was added to a CellTiter-Glo^{®} buffer. The mixture was mixed uniformly and equilibrated to room temperature, and then added at 100 µL/well to the wells to be tested. The resulting mixture was allowed to react at room temperature for 10 min and then subjected to full-wavelength detection.

The results for the inhibition of the enzymatic activity of the soluble recombinant CD73 antigen protein (ACRO, # CD3-H52H7-100 µg) by the antibody-drug conjugate 1, the antibody-drug conjugate 2, and the 900698 naked antibody are shown in FIG. 3 (represented as 1-3 in FIG. 3, respectively). The results show that the antibody-drug conjugate 1, the antibody-drug conjugate 2, and 900698 had comparable inhibitory effects on the enzymatic activity of the soluble recombinant CD73 protein, indicating that the ability of the 900698 naked antibody to inhibit the enzymatic activity of the recombinant CD73 antigen protein was not affected after conjugation to the SN38 small-molecule toxin.

### Example 4. Assay for Binding Affinity of Antibody-Drug Conjugates for Human and Monkey CD73 Antigen Proteins

The binding kinetics of the antibody-drug conjugate 1 and the antibody-drug conjugate 2 prepared in Example 1 and the 900698 naked antibody with a human CD73 protein (manufacturer: ACRO Biosystems, Cat. No. CD3-H52H7) and a monkey CD73 protein (manufacturer: Novoprotein, Cat. No. CI21) were assayed using Biacore (GE, model: T200), with an assay buffer of HBS-EP+ (pH 7.4) and a protein A chip (manufacturer: GE, Cat. No. 29-1275-56).

The temperature for the sample compartment and the flow path was set to 25 °C. The protein A chip captured each antigen-binding protein as a ligand, fixing about 100 RU. The human CD73 protein was diluted with the assay buffer to a series of concentrations of 50 nM, 25 nM, 12.5 nM, 6.25 nM, 3.12 nM, and 1.57 nM, serving as analytes, and the assay buffer was used as a 0 concentration control. At a flow rate of 30 µL/min, association was conducted for 120 s, and dissociation was conducted for 600 s. Glycine (10 mM, pH 1.5) was used for regeneration at a flow rate of 50 µL/min for 60 s in a multi-cycle kinetic assay. A 1:1 binding model was used, and the Fit Local mode was selected to determine the association rate constant (ka), dissociation rate constant (kd), and equilibrium dissociation constant (KD). The fitting results are shown in Table 1, indicating that all antigen-binding proteins bound to the human and monkey CD73 antigen proteins, and the binding affinity of the molecules, antibody-drug conjugate 1 and antibody-drug conjugate 2, for the human and CD73 proteins did not significantly differ from that of the 900698 naked antibody.

**Table 1. Assay results for the binding affinity for human and monkey CD73**

| Ligand | Antigen-binding protein | ka (1/Ms) | kd (1/s) | KD (M) |
|---|---|---|---|---|
| 900698 | Human CD73 | 1.47E+05 | 1.04E-04 | 7.11E-10 |
| Antibody-drug conjugate 1 | Human CD73 | 3.81E+05 | 1.29E-04 | 3.39E-10 |
| Antibody-drug conjugate 2 | Human CD73 | 6.43E+05 | 2.28E-04 | 3.54E-10 |
| 900698 | Monkey CD73 | 1.48E+05 | 1.80E-05 | 1.21E-10 |
| Antibody-drug conjugate 1 | Monkey CD73 | 5.50E+04 | 1.92E-06 | 3.49E-11 |
| Antibody-drug conjugate 2 | Monkey CD73 | 4.54E+05 | 1.14E-04 | 2.52E-10 |

### Example 5. Assay for Ability of Antibody-Drug Conjugates to Induce Endocytosis of CD73 Antigen on Cell Surface

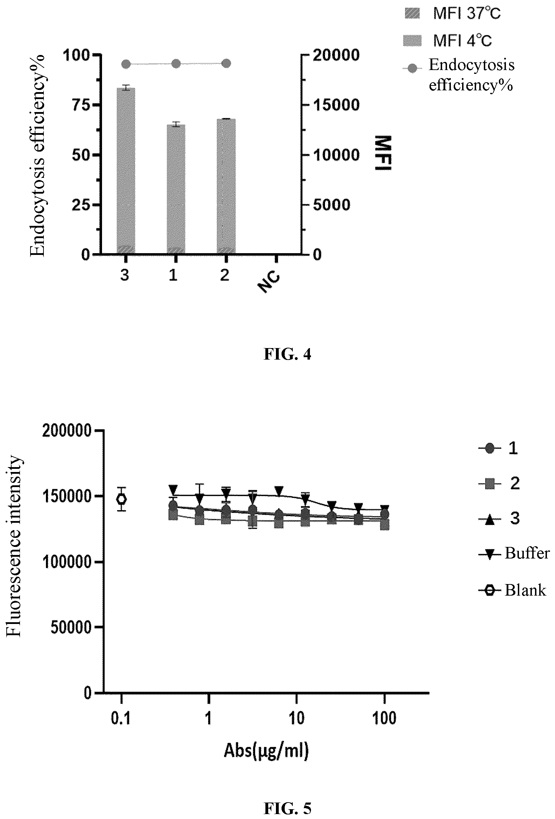

The antibody-drug conjugate 1 and the antibody-drug conjugate 2 prepared in Example 1 and the 900698 naked antibody were diluted to 1 µg/mL with a PBS solution, with 2 replicate wells set up for each concentration. Calu6 cells in the logarithmic growth phase were taken and prepared into a cell suspension at 2 × 10⁵ cells/mL. The cell suspension was then added at 100 µL (about 20000 cells) per well to a 96-well U-bottom plate and centrifuged at 300 g for 3 min, and then the supernatant was discarded. Then, the diluted antigen-binding proteins were each added at 100 µL/well to resuspend the cells, and the mixture was mixed uniformly and incubated at 4 °C for 30 min. After the incubation was completed, the mixture was centrifuged at 300 g for 3 min, and then the supernatant was discarded. The cells were then resuspended in 1% BSA, with half incubated at 37 °C and the other half at 4 °C, for an incubation time of 5 h. After the incubation was completed, the wells corresponding to the specified time were removed. The cells in these wells were centrifuged at 300 g for 3 min, and then the supernatant was discarded. PE anti-huIgG Fc (1 :200) was then added at 50 µL/well, and the mixture was mixed uniformly and allowed to react at 4 °C for 30 min. After the incubation was completed, the cells were washed twice and centrifuged at 300 g for 3 min, and the fluorescence intensity was detected by flow cytometry. The assay results for the endocytosis of CD73 on the cell surface induced by the anti-CD73 antigen-binding proteins are shown in Table 2 below and FIG. 4 (in FIG. 4, the antibody-drug conjugate 1, the antibody-drug conjugate 2, and 900698 are represented as 1-3, respectively, and NC indicates that only a 1% BSA solution was added without any antibody or antibody-drug conjugate). The results indicate that the ADC molecules, antibody-drug conjugate 1 and antibody-drug conjugate 2, had a similar ability to induce the endocytosis of CD73 on the cell surface as the 900698 naked antibody, and they performed excellently, with an endocytosis efficiency reaching up to about 95%.

**Table 2. Calculated values for the efficiency in inducing endocytosis of the CD73 antigen on the cell surface**

| Antigen-binding protein | 900698 | Antibody-drug conjugate 1 | Antibody-drug conjugate 2 |
|---|---|---|---|
| Endocytosis efficiency (%) | 95.42 | 95.64 | 95.80 |

### Example 6. Assay for Killing Ability of Antibody-Drug Conjugates Against Tumor Cells with Different Levels of CD73 Antigen Expression

The killing ability of the antibody-drug conjugate 1 and the antibody-drug conjugate 2 prepared in Example 1 and the 900698 naked antibody against different breast cancer cells (MDA-MB-231 and MCF-7) was assayed, where MDA-MB-231 highly expresses CD73 and MCF-7 does not express CD73.

The specific procedures were as follows: MDA-MB-231 cells and MCF-7 cells were prepared into cell suspensions at densities of 8E4 cells/mL and 7.5E5 cells/mL, respectively, which were then each added onto a 96-well cell culture plate at 100 µL per well. The 96-well cell culture plate was sealed with liquid and then incubated in an incubator at 37 °C with 5% CO₂ for 6 h. With an assay medium, the antibody-drug conjugate 1, the antibody-drug conjugate 2, and the 900698 naked antibody were each diluted to 400 µg/mL and then subjected to a 2-fold dilution to give 8 concentrations, resulting in a total of 9 concentrations. The same dilution procedure was performed for the Buffer solution as for the antibody-drug conjugate 1. The gradient dilution solutions were each added at 50 µL/well to the 96-well cell culture plate after the above incubation was completed. Each sample well was then supplemented with 50 µL of the corresponding assay medium. The 96-well cell culture plate was further incubated in an incubator at 37 °C with 5% CO₂ for another 5 days. After the incubation was completed, the 96-well plate was removed from the incubator, 100 µL of culture medium was aspirated from each well, and then the equilibrated Celltiter-Glo Luminescent Cell Viability Assay reagent (Promega, #G7573) was added at 100 µL/well. After shaking for reaction in the dark at room temperature for 10 min, 100 µL of culture medium was taken from each well, transferred to the corresponding well of a 96-well black plate, and then subjected to full-wavelength detection using a microplate reader.

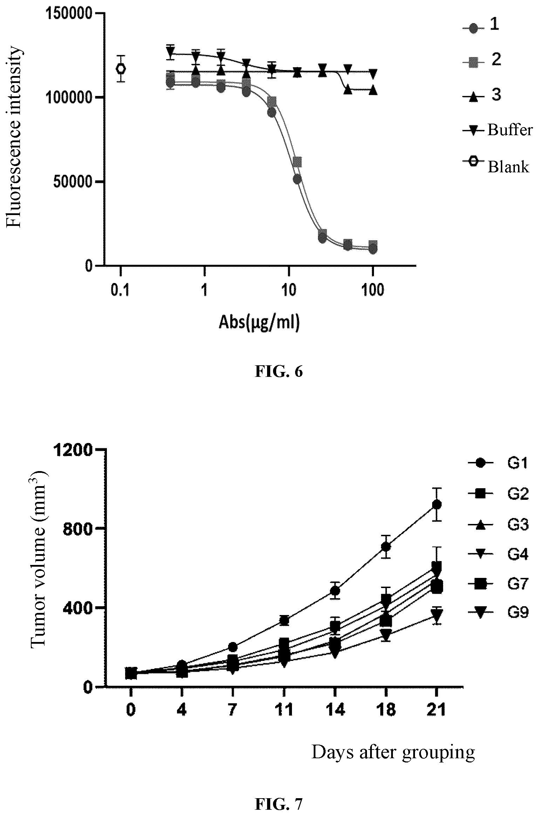

From the assay results in FIG. 5 (corresponding to MCF-7 cells) and FIG. 6 (corresponding to MDA-MB-231 cells), it can be seen that the antibody-drug conjugate 1 and the antibody-drug conjugate 2 had excellent killing effects on MDA-MB-231 cells highly expressing CD73, but had no killing effect on MCF-7 not expressing CD73, and that the naked antibody 900698 had no killing effect on both cell lines. The results indicate that the antibody-drug conjugate 1 and the antibody-drug conjugate 2 had high specificity in the killing of the tumor cells.

### Example 7. Assay for Antitumor Efficacy of Antibody-Drug Conjugates in BxPC3 CDX Pancreatic Cancer Mouse Efficacy Model

NPG mice were used to establish a BxPC-3 pancreatic cancer animal model for assaying the efficacy of the test antibodies. The BxPC-3 human pancreatic cancer cells used in this example were incubated in an RPMI-1640 medium supplemented with 10% FBS in an incubator at 37 °C with 5% CO₂. Before continuous cell culturing for ten generations, 100 µL of PBS containing about 1 × 10⁷ BxPC-3 cells was mixed uniformly with an equal volume of Matrigel. The mixture was then inoculated by subcutaneous injection into 18 NPG mice on the right side of the back near the axilla, with an inoculation volume of about 200 µL. The mice were anesthetized with 2%-5% isoflurane prior to the inoculation. On the day of inoculation, 1.0 × 10⁷ PBMCs (100 µL) were injected via the tail vein. When the tumors grew to an average size of about 50-80 mm³, the 18 tumor-bearing mice were randomly divided into 3 groups of 6 based on tumor volume and body weight. Administration was performed on the day of grouping, and the specific administration regimen is shown in Table 3 below. 900543 was used as a negative control antibody, which does not bind to the CD73 antigen. Oleclumab is an anti-CD73 monoclonal antibody developed by AstraZeneca, currently in Phase 2 clinical trials. Uliledlimab is an anti-CD73 monoclonal antibody developed by I-Mab Biopharma, currently in Phase 1 clinical trials. The amino acid sequence of the heavy chain of 900543 is as set forth in SEQ ID NO. 45, and the amino acid sequence of the light chain of 900543 is as set forth in SEQ ID NO. 46. The amino acid sequence of the heavy chain of Oleclumab is as set forth in SEQ ID NO. 47, and the amino acid sequence of the light chain of Oleclumab is as set forth in SEQ ID NO. 48. The amino acid sequence of the heavy chain of Uliledlimab is as set forth in SEQ ID NO. 49, and the amino acid sequence of the light chain of Uliledlimab is as set forth in SEQ ID NO. 50.

The results are shown in Table 3 below and FIG. 7, indicating that the antibody-drug conjugate 1 and the antibody-drug conjugate 2 could significantly inhibit the growth of BxPC-3 pancreatic cancer at an administration dose of 1 mg/kg, and their inhibitory effects on tumor growth were superior to those of the 900698 naked antibody and the positive control antibodies Oleclumab and Uliledlimab at an administration dose of 5 mg/kg, while the negative control antibody 900543 could not inhibit tumor growth.

**Table 3. Administration regimen for the BxPC3 CDX pancreatic cancer model**

| Group | Test sample | Number of animals | Administration dose (mg/kg) | Route of administration | Administration frequency | Number of doses |
|---|---|---|---|---|---|---|
| G1 | 900543 | 6 | 5 | i.p. | BIW | 6 |
| G2 | Oleclumab | 6 | 5 | i.p. | BIW | 6 |
| G3 | Uliledlimab | 6 | 5 | i.p. | BIW | 6 |
| G4 | 900698 | 6 | 5 | i.p. | BIW | 6 |
| G7 | Antibody-drug conjugate 1 | 6 | 1 | i.p. | BIW | 6 |
| G9 | Antibody-drug conjugate 2 | 6 | 1 | i.p. | BIW | 6 |

### Example 8. Assay for Antitumor Efficacy of Antibody-Drug Conjugates in MDA-MB-231 Triple-Negative Breast Mouse CDX Efficacy Model

NPG mice were used to establish an MDA-MB-231 triple-negative breast cancer animal model for assaying the efficacy of the test antibodies. The MDA-MB-231 human breast cancer cells used in this example were incubated in an L-15 medium supplemented with 10% FBS in an incubator at 37 °C without CO₂. Before continuous cell culturing for ten generations, about 5.0 × 10⁶ MDA-MB-231 cells were suspended in 100 µL of PBS and then mixed uniformly with an equal volume of Matrigel. The mixture was then inoculated by subcutaneous injection into NPG humanized mice on the right side of the back near the axilla, with an inoculation volume of about 200 µL. The mice were anesthetized with 2%-5% isoflurane prior to the inoculation. On the day of inoculation, 1.0 × 10⁷ PBMCs (100 µL) were injected via the tail vein. When the tumors grew to an average size of about 50-80 mm³, the 18 tumor-bearing mice were randomly divided into 7 groups of 6 based on tumor volume and body weight. Administration was performed on the day of grouping, and the specific administration regimen is shown in Table 4 below. 900201 was used as a negative control antibody, which does not bind to the CD73 antigen. Oleclumab is an anti-CD73 monoclonal antibody developed by AstraZeneca, currently in Phase 2 clinical trials. The amino acid sequence of the heavy chain of 900201 is as set forth in SEQ ID NO. 43, and the amino acid sequence of the light chain of 900201 is as set forth in SEQ ID NO. 44.

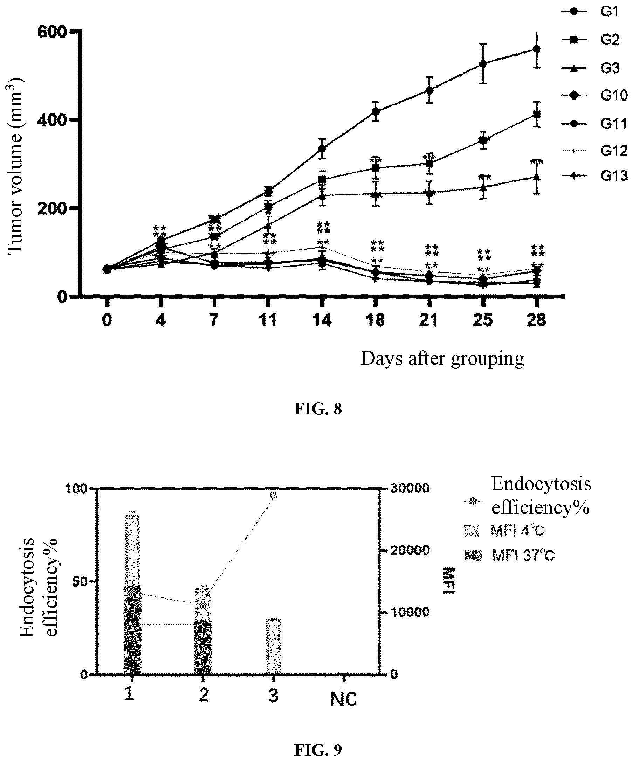

The results are shown in Table 4 below and FIG. 8, indicating that the antibody-drug conjugate 1 and the antibody-drug conjugate 2 could significantly inhibit the growth of MDA-MB-231 breast cancer, and their anti-tumor effects were positively correlated with the administration dose. Meanwhile, their inhibitory effects on tumor growth were significantly superior to those of the 900698 naked antibody and the positive control antibody Oleclumab at an administration dose of 5 mg/kg, while the negative control antibody 900201 could not inhibit the tumor growth.

**Table 4. Administration regimen for the MDA-MB-231 triple-negative breast cancer CDX model**

| Group | Test sample | Number of animals | Administration dose (mg/kg) | Route of administration | Administration frequency | Number of doses |
|---|---|---|---|---|---|---|
| G1 | 900201 | 6 | 5 | i.p. | BIW | 8 |
| G2 | Oleclumab | 6 | 5 | i.p. | BIW | 8 |
| G3 | 900698 | 6 | 5 | i.p. | BIW | 8 |
| G10 | Antibody-drug conjugate 1 | 6 | 2.5 | i.p. | BIW | 8 |
| G11 | Antibody-drug conjugate 1 | 6 | 5 | i.p. | BIW | 8 |
| G12 | Antibody-drug conjugate 2 | 6 | 2.5 | i.p. | BIW | 8 |
| G13 | Antibody-drug conjugate 2 | 6 | 5 | i.p. | BIW | 8 |

### Example 9. Selection of Antibodies for Preparing Antibody-Drug Conjugates

The endocytosis efficiency of monoclonal antibodies 900698 and 900725 was compared through an endocytosis assay, with the procedures as follows: Anti-CD73 antigen-binding proteins such as the CD73 positive control antibody BMS-986179, 900698, and 900725 were diluted to 1 µg/mL with a PBS solution, with 2 replicate wells set up for each concentration. Calu6 cells in the logarithmic growth phase were taken and prepared into a cell suspension at 2 × 10⁵ cells/mL. The cell suspension was then added at 100 µL (about 20000 cells) per well to a 96-well U-bottom plate and centrifuged at 300 g for 3 min, and then the supernatant was discarded. Then, the diluted antigen-binding proteins were each added at 100 µL/well to resuspend the cells, and the mixture was mixed uniformly and incubated at 4 °C for 30 min. After the incubation was completed, the mixture was centrifuged at 300 g for 3 min, and then the supernatant was discarded. The cells were then resuspended in 1% BSA, with half incubated at 37 °C and the other half at 4 °C, for an incubation time of 5 h. After the incubation was completed, the wells corresponding to the specified time were removed. The cells in these wells were centrifuged at 300 g for 3 min, and then the supernatant was discarded. PE anti-huIgG Fc (1:200) was then added at 50 µL/well, and the mixture was mixed uniformly and allowed to react at 4 °C for 30 min. After the incubation was completed, the cells were washed twice and centrifuged at 300 g for 3 min, and the fluorescence intensity was detected by flow cytometry. The assay results for the endocytosis of CD73 on the cell surface induced by the anti-CD73 antigen-binding proteins are shown in Table 5 below and FIG. 9. The results indicate that the efficiency of the anti-CD73 antibody 900725 in inducing endocytosis of CD73 on the cell surface (37.49%) was similar to that of the positive control antibody BMS-986179 (44.18%), while the endocytosis efficiency of 900698 was significantly higher than that of both 900725 and BMS-986179, reaching up to about 96%. 34%. In FIG. 9, the results for BMS-986179, 900725, and 900698 are represented as 1-3, respectively, and NC indicates the result for the condition that only a 1% BSA solution was added without any antibody or antibody-drug conjugate. These results indicate that 900698 is more suitable for developing ADC drugs compared to 900725. The amino acid sequence of the heavy chain of BMS-986179 is as set forth in SEQ ID NO. 51, and the amino acid sequence of the light chain of BMS-986179 is as set forth in SEQ ID NO. 52.

**Table 5. Calculated values for the endocytosis efficiency of 900698 and 900725**

| Antibody | BMS-986179 | 900725 | 900698 |
|---|---|---|---|
| 4°C | 25710 | 13911.5 | 8937 |
| 37°C | 14364.5 | 8707 | 359 |
| Endocytosis efficiency% | 44.18 | 37.49 | 96.34 |

### Example 10. Preparation of Antibody-Drug Conjugates and Anti-Tumor Effect

In order to verify the anti-tumor effects of ADCs prepared by conjugating the anti-CD73 antibody 900698 to different linkers and payloads, a tubulin polymerization inhibitor MMAE small molecule was conjugated to the 900698 antibody using cysteine conjugation through two different linkers, resulting in two ADC molecules: 900698-MC-VC-PAB-MMAE (hereinafter abbreviated as antibody-drug conjugate 3) and 900698-PY-VC-PAB-MMAE (hereinafter abbreviated as antibody-drug conjugate 4).

The chemical structural formulas of MC-VC-PAB-MMAE and PY-VC-PAB-MMAE are shown as follows, and the mAb is the 900698 antibody. In the antibody-drug conjugate 3 and the antibody-drug conjugate 4, the 900698 antibody was linked to MC-VC-PAB-MMAE or PY-VC-PAB-MMAE, as shown in FIG. 1A.

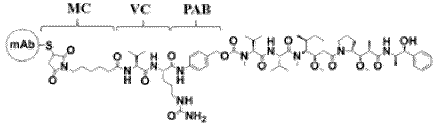

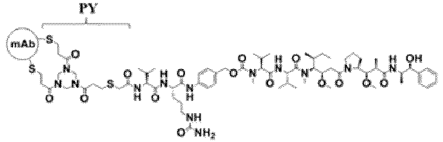

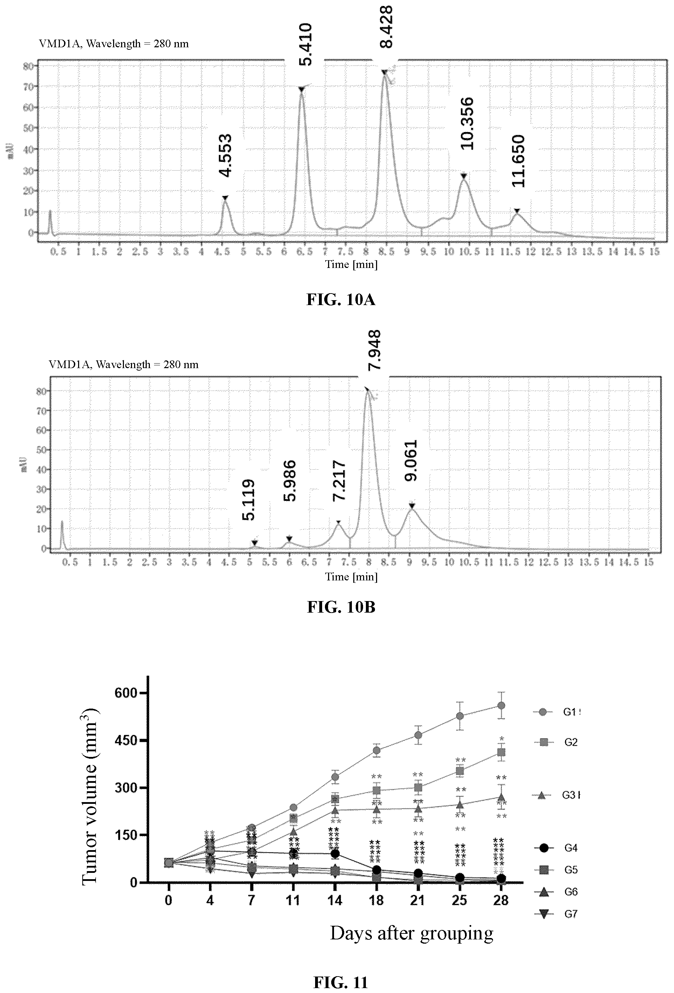

In addition, the drug-to-antibody ratios (DAR values) of the antibody-drug conjugate 3 and the antibody-drug conjugate 4 were determined by hydrophobic chromatography, and the DAR values and HPLC chromatograms (at a wavelength of 280 nm) thereof are shown in Table 6 below and FIGs. 10A-10B.

**Table 6. DAR values and purity measurements**

| Sample name | Concentration (mg/mL) | DAR (RP-HPLC) | Monomer purity (%) |
|---|---|---|---|
| Antibody-drug conjugate 3 | 6.6 | 4.1 | 99.6 |
| Antibody-drug conjugate 4 | 8.4 | 4.21 | 99.1 |

To compare the *in vivo* anti-tumor effects of the antibody-drug conjugate 3 and the antibody-drug conjugate 4, we conducted an assay using an NPG mouse model inoculated with the human triple-negative breast cancer cell line MDA-MB-231 that highly expresses the CD73 antigen.

The specific procedures were as follows: The MDA-MB-231 human breast cancer cells used in this assay were incubated in an L-15 medium supplemented with 10% FBS in an incubator at 37 °C without CO₂. Before continuous cell culturing for ten generations, about 5.0 × 10⁶ MDA-MB-231 cells were suspended in 100 µL of PBS and then mixed uniformly with an equal volume of Matrigel. The mixture was then inoculated by subcutaneous injection into NPG humanized mice on the right side of the back near the axilla, with an inoculation volume of about 200 µL. The mice were anesthetized with 2%-5% isoflurane prior to the inoculation. On the day of inoculation, 1.0 × 10⁷ PBMCs (100 µL) were injected via the tail vein. When the tumors grew to an average size of about 50-80 mm³, the 60 tumor-bearing mice were randomly divided into 7 groups of 6 based on tumor volume and body weight. Administration was performed on the day of grouping, and the specific administration regimen is shown in Table 7 below. Twenty-eight days after administration, the mice were dissected, and the tumor volume and size were measured to assess the anti-tumor effects. The results are shown in FIG. 11. From the results, it can be seen that the antibody-drug conjugate 3 and the antibody-drug conjugate 4 both had better anti-tumor effects compared to the monoclonal antibodies, and the anti-tumor effects of the two ADCs were comparable.

**Table 7. Administration regimen for the triple-negative breast cancer model**

| Group | Test sample | Number of animals | Administration dose (mg/kg) | Route of administration | Administration frequency | Number of doses |
|---|---|---|---|---|---|---|
| G1 | 900201 | 6 | 5 | i.p. | BIW | 8 |
| G2 | Oleclumab | 6 | 5 | i.p. | BIW | 8 |
| G3 | 900698 | 6 | 5 | i.p. | BIW | 8 |
| G4 | Antibody-drug conjugate 3 | 6 | 2.5 | i.p. | BIW | 8 |
| G5 | Antibody-drug conjugate 3 | 6 | 5 | i.p. | BIW | 8 |
| G6 | Antibody-drug conjugate 4 | 6 | 2.5 | i.p. | BIW | 8 |
| G7 | Antibody-drug conjugate 4 | 6 | 5 | i.p. | BIW | 8 |

The foregoing detailed description is provided by way of illustration and example, and is not intended to limit the scope of the appended claims. Various variants of the embodiments currently listed in the present application will be apparent to those of ordinary skill in the art, and are intended to be within the scope of the appended claims and equivalents thereof.

## Claims

1. An antibody-drug conjugate, comprising a CD73-targeting antibody or an antigen-binding fragment thereof, wherein the CD73-targeting antibody or the antigen-binding fragment thereof comprises HCDR3, the HCDR3 comprising the amino acid sequence as set forth in SEQ ID NO: 3.

2. The antibody-drug conjugate according to claim 1, wherein the CD73-targeting antibody or the antigen-binding fragment thereof comprises HCDR2, the HCDR2 comprising the amino acid sequence as set forth in SEQ ID NO: 2.

3. The antibody-drug conjugate according to any one of claims 1-2, wherein the CD73-targeting antibody or the antigen-binding fragment thereof comprises HCDR1, the HCDR1 comprising the amino acid sequence as set forth in SEQ ID NO: 1.

4. The antibody-drug conjugate according to any one of claims 1-3, wherein the CD73-targeting antibody or the antigen-binding fragment thereof comprises LCDR3, the LCDR3 comprising the amino acid sequence as set forth in SEQ ID NO: 6.

5. The antibody-drug conjugate according to any one of claims 1-4, wherein the CD73-targeting antibody or the antigen-binding fragment thereof comprises LCDR2, the LCDR2 comprising the amino acid sequence as set forth in SEQ ID NO: 5.

6. The antibody-drug conjugate according to any one of claims 1-5, wherein the CD73-targeting antibody or the antigen-binding fragment thereof comprises LCDR1, the LCDR1 comprising the amino acid sequence as set forth in SEQ ID NO: 4.

7. The antibody-drug conjugate according to any one of claims 3-6, wherein the CD73-targeting antibody or the antigen-binding fragment thereof comprises H-FR1, the C-terminus of the H-FR1 being linked directly or indirectly to the N-terminus of the HCDR1, and the H-FR1 comprising the amino acid sequence as set forth in SEQ ID NO: 7, SEQ ID NO: 15, or SEQ ID NO: 19.

8. The antibody-drug conjugate according to any one of claims 2-7, wherein the CD73-targeting antibody or the antigen-binding fragment thereof comprises H-FR2, the H-FR2 being located between the HCDR1 and the HCDR2, and the H-FR2 comprising the amino acid sequence as set forth in SEQ ID NO: 8, SEQ ID NO: 16, or SEQ ID NO: 21.

9. The antibody-drug conjugate according to any one of claims 2-8, wherein the CD73-targeting antibody or the antigen-binding fragment thereof comprises H-FR3, the H-FR3 being located between the HCDR2 and the HCDR3, and the H-FR3 comprising the amino acid sequence as set forth in SEQ ID NO: 9, SEQ ID NO: 17, SEQ ID NO: 20, or SEQ ID NO: 22.

10. The antibody-drug conjugate according to any one of claims 1-9, wherein the CD73-targeting antibody or the antigen-binding fragment thereof comprises H-FR4, the N-terminus of the H-FR4 being linked to the C-terminus of the HCDR3, and the H-FR4 comprising the amino acid sequence as set forth in SEQ ID NO: 10 or SEQ ID NO: 18.

11. The antibody-drug conjugate according to any one of claims 6-10, wherein the CD73-targeting antibody or the antigen-binding fragment thereof comprises L-FR1, the C-terminus of the L-FR1 being linked directly or indirectly to the N-terminus of the LCDR1, and the L-FR1 comprising the amino acid sequence as set forth in SEQ ID NO: 11 or SEQ ID NO: 23.

12. The antibody-drug conjugate according to any one of claims 6-11, wherein the CD73-targeting antibody or the antigen-binding fragment thereof comprises L-FR2, the L-FR2 being located between the LCDR1 and the LCDR2, and the L-FR2 comprising the amino acid sequence as set forth in SEQ ID NO: 12 or SEQ ID NO: 24.

13. The antibody-drug conjugate according to any one of claims 5-12, wherein the CD73-targeting antibody or the antigen-binding fragment thereof comprises L-FR3, the L-FR3 being located between the LCDR2 and the LCDR3, and the L-FR3 comprising the amino acid sequence as set forth in SEQ ID NO: 13, SEQ ID NO: 25, SEQ ID NO: 27, or SEQ ID NO: 28.

14. The antibody-drug conjugate according to any one of claims 4-13, wherein the CD73-targeting antibody or the antigen-binding fragment thereof comprises L-FR4, the N-terminus of the L-FR4 being linked to the C-terminus of the LCDR3, and the L-FR4 comprising the amino acid sequence as set forth in SEQ ID NO: 14 or SEQ ID NO: 26.

15. The antibody-drug conjugate according to any one of claims 1-14, wherein the CD73-targeting antibody or the antigen-binding fragment thereof comprises HCDR1, HCDR2, HCDR3, LCDR1, LCDR2, and LCDR3, wherein the HCDR1 comprises the amino acid sequence as set forth in SEQ ID NO: 1, the HCDR2 comprises the amino acid sequence as set forth in SEQ ID NO: 2, the HCDR3 comprises the amino acid sequence as set forth in SEQ ID NO: 3, the LCDR1 comprises the amino acid sequence as set forth in SEQ ID NO: 4, the LCDR2 comprises the amino acid sequence as set forth in SEQ ID NO: 5, and the LCDR3 comprises the amino acid sequence as set forth in SEQ ID NO: 6.

16. The antibody-drug conjugate according to any one of claims 1-15, wherein the CD73-targeting antibody or the antigen-binding fragment thereof comprises VH, the VH comprising the amino acid sequence as set forth in SEQ ID NO: 29, SEQ ID NO: 31, SEQ ID NO: 33, or SEQ ID NO: 35.

17. The antibody-drug conjugate according to any one of claims 1-16, wherein the CD73-targeting antibody or the antigen-binding fragment thereof comprises VL, the VL comprising the amino acid sequence as set forth in SEQ ID NO: 30, SEQ ID NO: 32, SEQ ID NO: 34, or SEQ ID NO: 36.

18. The antibody-drug conjugate according to any one of claims 1-17, wherein the CD73-targeting antibody or the antigen-binding fragment thereof comprises any one of the groups of VH and VL selected from:
1) the VH comprising the amino acid sequence as set forth in SEQ ID NO: 29 and the VL comprising the amino acid sequence as set forth in SEQ ID NO: 30;
2) the VH comprising the amino acid sequence as set forth in SEQ ID NO: 31 and the VL comprising the amino acid sequence as set forth in SEQ ID NO: 32;
3) the VH comprising the amino acid sequence as set forth in SEQ ID NO: 33 and the VL comprising the amino acid sequence as set forth in SEQ ID NO: 34;
4) the VH comprising the amino acid sequence as set forth in SEQ ID NO: 35 and the VL comprising the amino acid sequence as set forth in SEQ ID NO: 36;
5) the VH comprising the amino acid sequence as set forth in SEQ ID NO: 33 and the VL comprising the amino acid sequence as set forth in SEQ ID NO: 36; and
6) the VH comprising the amino acid sequence as set forth in SEQ ID NO: 35 and the VL comprising the amino acid sequence as set forth in SEQ ID NO: 34.

19. The antibody-drug conjugate according to any one of claims 1-18, wherein the CD73-targeting antibody or the antigen-binding fragment thereof comprises an antibody heavy chain constant region.

20. The antibody-drug conjugate according to claim 19, wherein the antibody heavy chain constant region is derived from a human antibody heavy chain constant region.

21. The antibody-drug conjugate according to any one of claims 19-20, wherein the antibody heavy chain constant region is derived from a human IgG heavy chain constant region.

22. The antibody-drug conjugate according to any one of claims 19-21, wherein the antibody heavy chain constant region is derived from a human IgG1 heavy chain constant region.

23. The antibody-drug conjugate according to any one of claims 1-22, wherein the CD73-targeting antibody or the antigen-binding fragment thereof comprises an antibody light chain constant region.

24. The antibody-drug conjugate according to claim 23, wherein the antibody light chain constant region is derived from a human Igκ constant region.

25. The antibody-drug conjugate according to any one of claims 1-24, wherein the CD73-targeting antibody or the antigen-binding fragment thereof comprises an antibody or an antigen-binding fragment thereof.

26. The antibody-drug conjugate according to claim 25, wherein the antigen-binding fragment comprises a Fab, a Fab', an Fv fragment, an F(ab')₂, an F(ab)₂, an scFv, a di-scFv, and/or a dAb.

27. The antibody-drug conjugate according to any one of claims 25-26, wherein the antibody is selected from one or more of the group consisting of: a monoclonal antibody, a chimeric antibody, a humanized antibody, and a fully human antibody.

28. The antibody-drug conjugate according to any one of claims 1-27, wherein the CD73 comprises human CD73 and/or monkey CD73.

29. The antibody-drug conjugate according to any one of claims 1-28, being capable of preventing or reducing activation of the CD73.

30. The antibody-drug conjugate according to any one of claims 1-29, being capable of inhibiting the enzymatic activity of the CD73.

31. The antibody-drug conjugate according to any one of claims 1-30, being capable of inducing endocytosis of the CD73 on a cell surface.

32. The antibody-drug conjugate according to any one of claims 1-31, comprising a structure represented by formula 1:
M-(L1)ₐ-(L2)_{b}-D (formula 1),
wherein M represents the CD73-targeting antibody or the antigen-binding fragment thereof according to any one of claims 1-31;
L1 represents a linker linking to M, and L2 represents a linker linking to D;
a and b are each independently selected from 0-10;
D represents a drug.

33. The antibody-drug conjugate according to claim 32, wherein the L1 and/or the L2 are/is selected from the group consisting of: a cleavable linker, a non-cleavable linker, a hydrophilic linker, a hydrophobic linker, a charged linker, an uncharged linker, and a dicarboxylic acid-based linker.

34. The antibody-drug conjugate according to any one of claims 32-33, wherein the L1 is linked to the M by a sulfhydryl group, an azido group, or an amide group on the M.

35. The antibody-drug conjugate according to any one of claims 32-34, wherein the L1 is capable of reacting with the sulfhydryl group to achieve linkage.

36. The antibody-drug conjugate according to any one of claims 32-35, wherein the L1 is selected from the group consisting of: 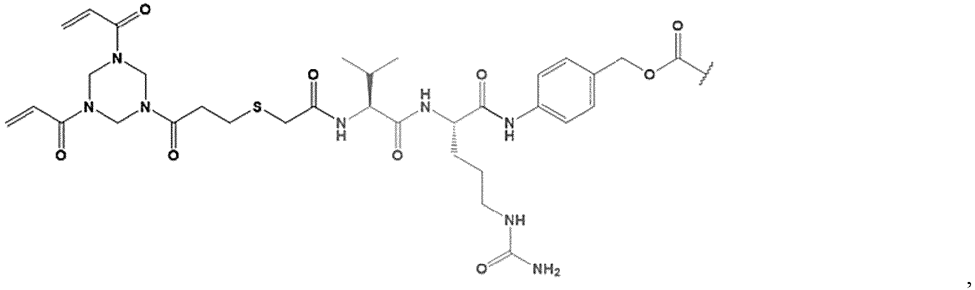 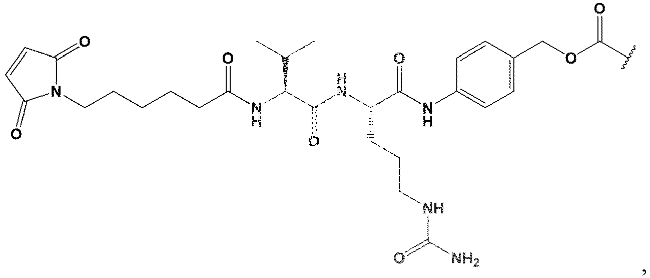 and

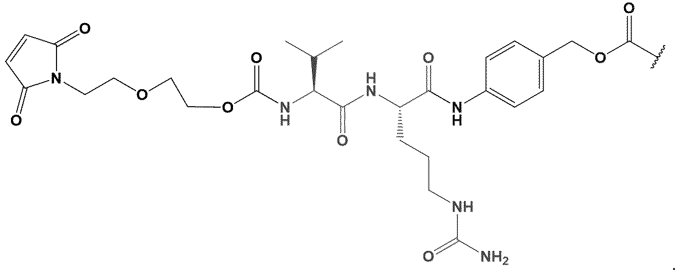

37. The antibody-drug conjugate according to any one of claims 32-36, wherein the L2 is selected from the group consisting of: a polypeptide, N-succinimidyl-3-(2-pyridyldithio)propionate (SPDP), N-succinimidyl 4-(2-pyridyldithio)pentanoate (SPP), N-succinimidyl 4-(2-pyridyldithio)butyrate (SPDB), N-succinimidyl-4-(2-pyridyldithio)-2-sulfobutyrate (sulfo-SPDB), N-succinimidyl iodoacetate (SIA), N-succinimidyl (4-iodoacetyl)aminobenzoate (SIAB), maleimide PEG NHS, succinimidyl 4-(N-maleimidomethyl)cyclohexane-1-carboxylate (SMCC), sulfosuccinimidyl 4-(N-maleimidomethyl)cyclohexane-1-carboxylate (sulfo-SMCC), and 2,5-dioxopyrrolidin-1-yl 17-(2,5-dioxo-2,5-dihydro-1H-pyrrol-1-yl)-5,8, 11, 14-tetraoxo-4,7, 10, 13-tetraazaoctadecan-1-oate (CX1-1).

38. The antibody-drug conjugate according to any one of claims 32-37, wherein the L2 is:

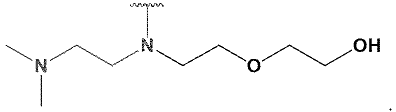

39. The antibody-drug conjugate according to any one of claims 32-38, wherein the L1-L2 is selected from the group consisting of: 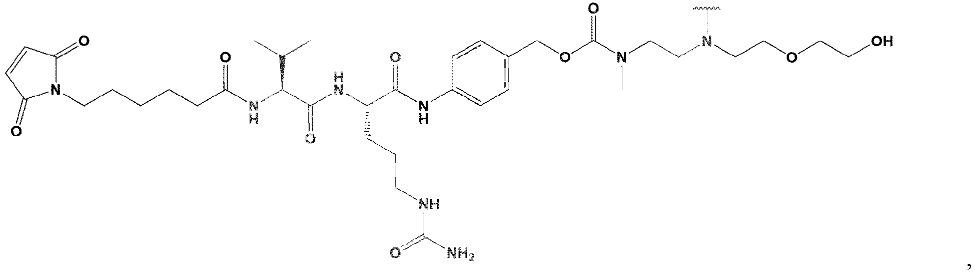 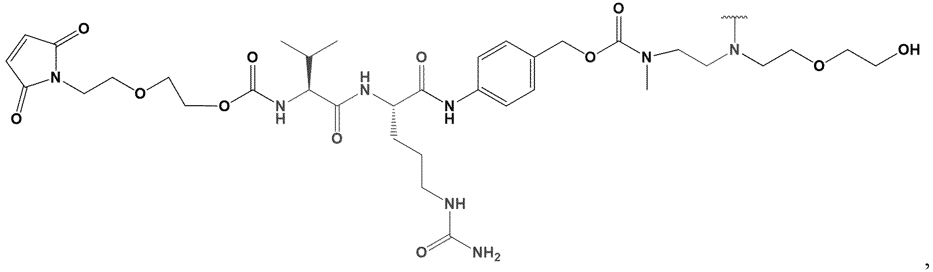 and

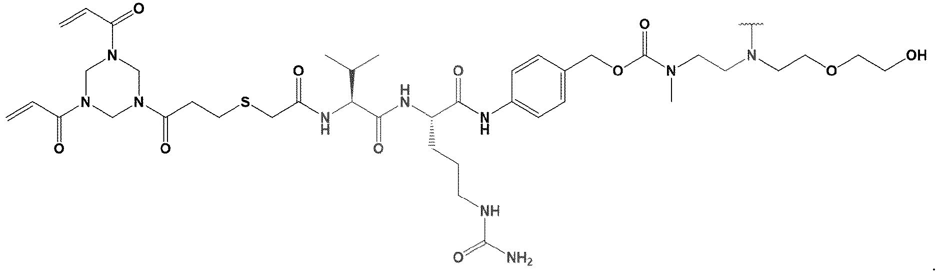

40. The antibody-drug conjugate according to any one of claims 32-39, wherein the drug has an ability to kill tumor cells, and/or an ability to inhibit tumor cell growth.

41. The antibody-drug conjugate according to any one of claims 32-40, wherein the drug comprises a small-molecule drug.

42. The antibody-drug conjugate according to any one of claims 32-41, wherein the drug is selected from the group consisting of: a V-ATPase inhibitor, a Bcl2 inhibitor, an MCL1 inhibitor, an HSP90 inhibitor, an IAP inhibitor, an mTor inhibitor, a microtubule stabilizer, a microtubule destabilizer, auristatin, dolastatin, a maytansinoid, MetAP (methionine aminopeptidase), an inhibitor of nuclear export of protein CRM1, a DPPIV inhibitor, a proteasome inhibitor, an inhibitor of phosphoryl transfer reactions in mitochondria, a protein synthesis inhibitor, a CDK2 inhibitor, a CDK9 inhibitor, a kinesin inhibitor, an HDAC inhibitor, a DNA damaging agent, a DNA alkylating agent, a DNA intercalator, a DNA minor groove binder, a DHFR inhibitor, a nucleoside analog, an HD AC inhibitor, an anthracycline, a NAMPT inhibitor, SN-38 or a derivative thereof, etoposide phosphate, nitrogen mustard, a proteasome inhibitor, a cytokine, a tubulysin B analog, and a Toll-like receptor agonist.

43. The antibody-drug conjugate according to any one of claims 32-42, wherein the drug is selected from the group consisting of: DM1, exatecan, Dxd, MMAE, calicheamicin, anthracyclin-5G, DM4, microtubule inhibitor SHR153024, PNU-159682, eribulin, Tub196, Duo5 toxin, SN38, or derivatives thereof.

44. The antibody-drug conjugate according to any one of claims 32-43, wherein (L1)ₐ-(L2)_{b}-D has a structure selected from the group consisting of: 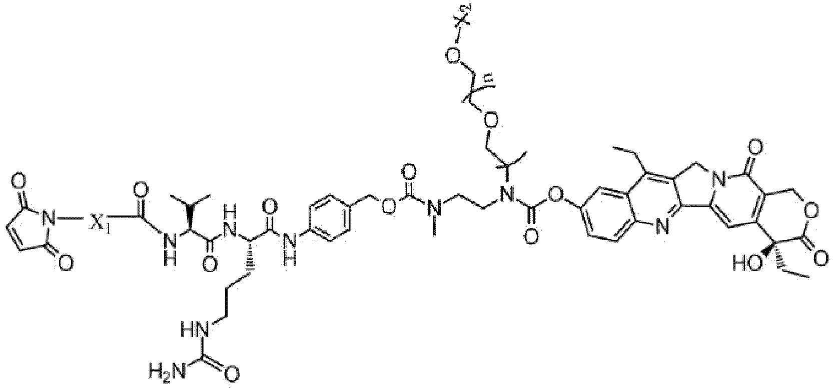 wherein
X₁ is an alkane chain or a PEG chain, and
X₂ is H or -C(O)NR¹R², and
R¹ is selected from hydrogen, halogen, hydroxyl, substituted or unsubstituted C₁₋₆ alkyl, substituted or unsubstituted C₁₋₆ hydroxyalkyl, substituted or unsubstituted C₁₋₆ aminoalkyl, substituted or unsubstituted C₁₋₆ alkoxy, substituted or unsubstituted C₁₋₆ alkanoyl, and a substituted or unsubstituted C₁₋₆ alkyl aldehyde group;
R² is selected from hydrogen and substituted or unsubstituted C₁₋₆ alkyl; each substituted C₁₋₆ alkyl is optionally substituted with 1-5 substituents independently selected from: halogen, hydroxyl, amino, carbonyl, carboxyl, 5- to 10-membered heteroaryl, and C₁₋₆ haloalkyl, wherein the 5- to 10-membered heteroaryl has 1-3 heteroatoms selected from oxygen, nitrogen, and sulfur; n is an integer greater than or equal to 1 and less than or equal to 20.

45. The antibody-drug conjugate according to any one of claims 32-44, wherein (L1)ₐ-(L2)_{b}-D has a structure selected from the group consisting of: 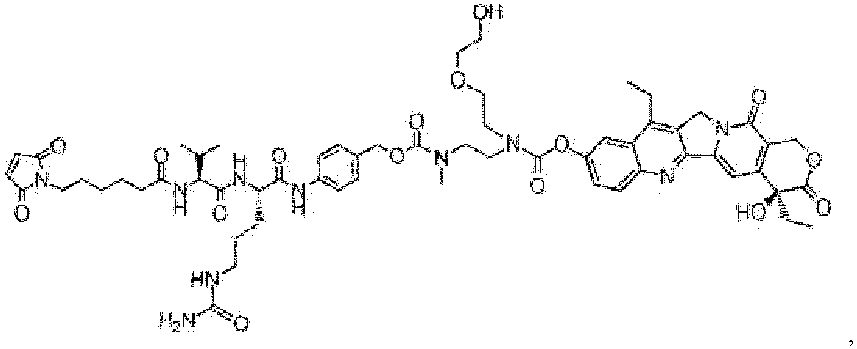 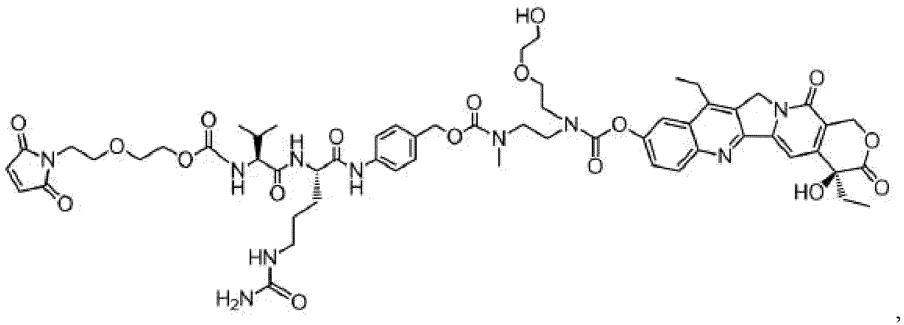 and

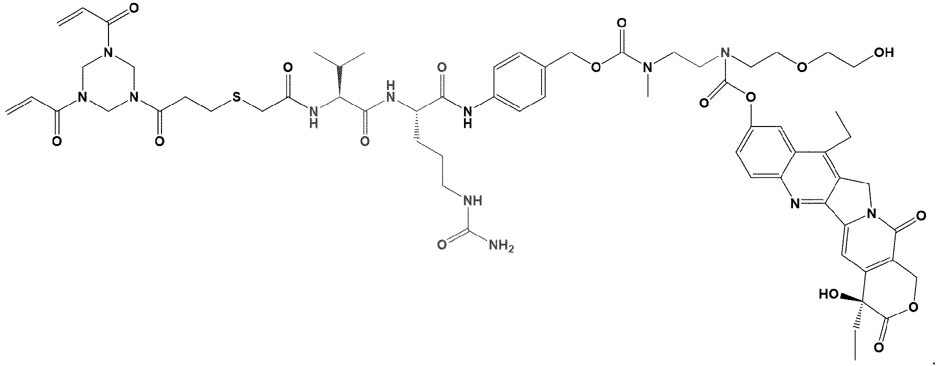

46. The antibody-drug conjugate according to any one of claims 32-45, having a structure selected from the group consisting of: 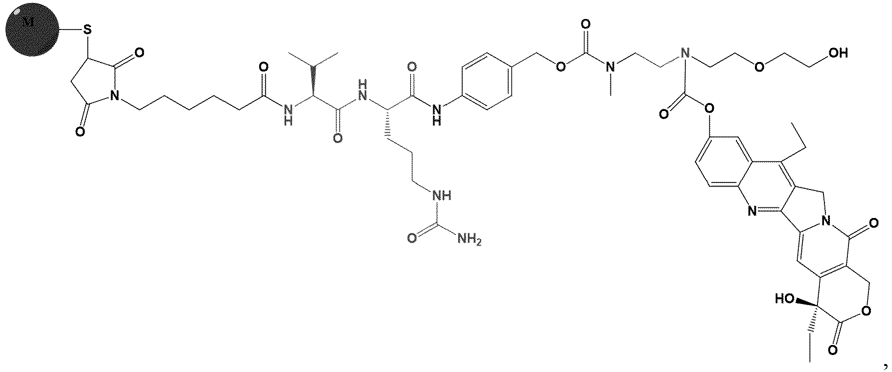 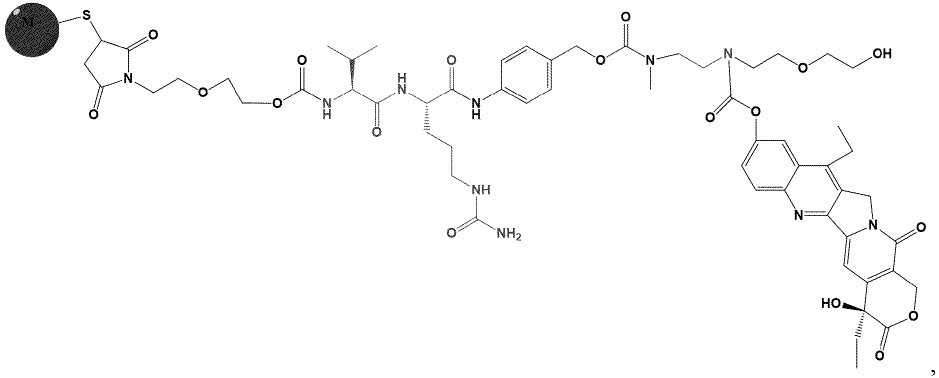 and

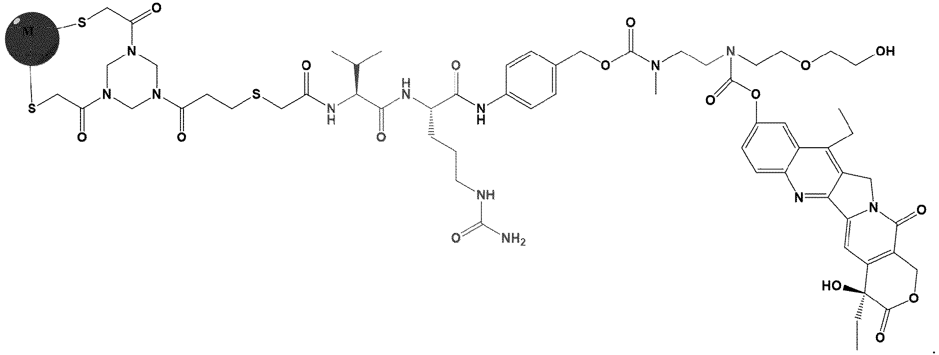

47. The antibody-drug conjugate according to any one of claims 1-46, having a drug-to-antibody ratio of about 1 to about 8.

48. A compound for preparing the antibody-drug conjugate according to any one of claims 1-47, having a structure represented by formula 2:
M-(L1)ₐ (formula 2), wherein M represents the CD73-targeting antibody or the antigen-binding fragment thereof according to any one of claims 1-31;
L1 represents a linker linking to M;
a is selected from 0-10.

49. The compound according to claim 48, wherein the L1 is selected from the group consisting of: a cleavable linker, a non-cleavable linker, a hydrophilic linker, a hydrophobic linker, a charged linker, an uncharged linker, and a dicarboxylic acid-based linker.

50. The compound according to any one of claims 48-49, wherein the L1 is linked to the M by a sulfhydryl group, an azido group, or an amide group on the M.

51. The compound according to any one of claims 48-50, wherein the L1 is capable of reacting with the sulfhydryl group to achieve linkage.

52. The compound according to any one of claims 48-51, wherein the L1 is selected from the group consisting of: 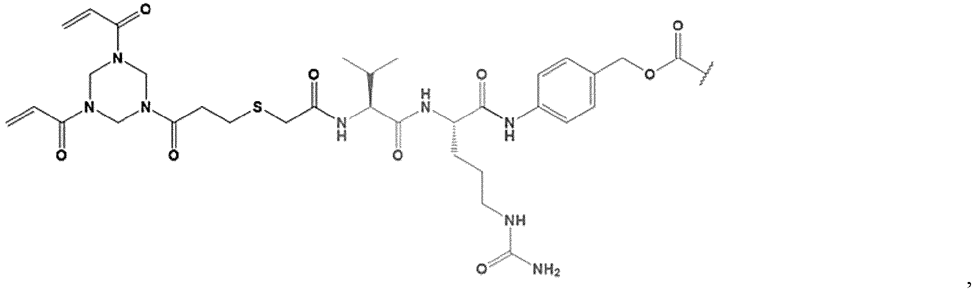 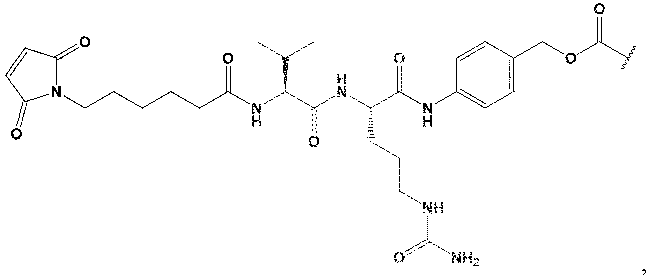 and

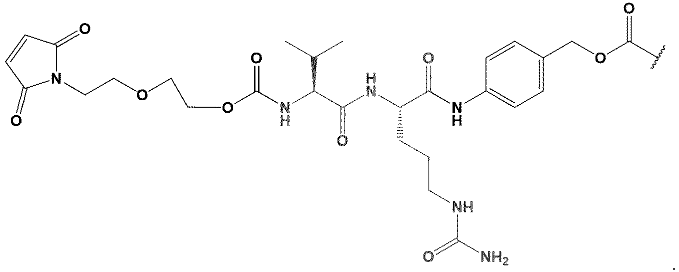

53. A method for preparing the antibody-drug conjugate according to any one of claims 1-47, comprising the following step:
contacting the compound according to any one of claims 48-52 with the drug according to any one of claims 40-43.

54. A pharmaceutical composition, comprising the antibody-drug conjugate according to any one of claims 1-47 and optionally a pharmaceutically acceptable carrier.

55. A method for modulating a tumor microenvironment in a subject, comprising the following step: administering to the subject the antibody-drug conjugate according to any one of claims 1-47 or the pharmaceutical composition according to claim 54.

56. A method for modulating an immune response in a subject, comprising the following step: administering to the subject the antibody-drug conjugate according to any one of claims 1-47 or the pharmaceutical composition according to claim 54.

57. Use of the antibody-drug conjugate according to any one of claims 1-47 or the pharmaceutical composition according to claim 54 in the preparation of a medicament, wherein the medicament is capable of preventing and/or treating a tumor.

58. The use according to claim 57, wherein the tumor comprises a solid tumor and/or a non-solid tumor.

59. The use according to any one of claims 57-58, wherein the tumor comprises a CD73-mediated tumor.

60. The use according to any one of claims 57-59, wherein the tumor comprises pancreatic cancer and/or breast cancer.
